# EUROPEAN PATENT SPECIFICATION

(11) **EP 1 280 940 B1**
(45) Date of publication and mention of the grant of the patent: **12.11.2008**
(21) Application number: 01943378.8
(22) Date of filing: 16.05.2001
(51) Int. Cl.: C12Q 1/68

(54) **COMPOSITIONS AND METHODS FOR GENETIC ANALYSES**
ZUSAMMENZETZUNGEN UND VERFAHREN ZUR GENETISCHEN ANALYSE
COMPOSITIONS ET PROCEDES POUR ANALYSES GENETIQUES

(30) Priority: 18.05.2000 EP 00401356
(43) Date of publication of application: 05.02.2003
(73) Proprietor: CENTRE NATIONAL DE LA RECHERCHE SCIENTIFIQUE (CNRS), 75794 Paris Cedex 16 (FR)
(72) Inventor: DUMAS, Sylvie, F-75006 Paris (FR); SALIN, Hélène, F-75013 Paris (FR); MALLET, Jacques, F-75013 Paris (FR)
(74) Representative: Becker, Philippe
(86) International application number: PCT/EP2001/005558
(87) International publication number: WO 2001/088184

(56) References cited:
- WO-A-97/40189
- WO-A-98/38329
- FR-A- 2 772 484
- US-A- 5 830 645
- US-A- 5 928 870
- US-A- 5 955 272
- LANIECE P ET AL: "A new high resolution for the quantitative analysis of radiolaelled molecules in tissue section" JOURNAL OF NEUROSCIENCE METHODS, vol. 86, 1998, pages 1-5, XP001024001
- PI¹TU G ET AL: "The gene express image knowledge base of the human transcriptome" GENOME RESEARCH, vol. 9, no. 2, - February 1999 (1999-02) pages 195-209, XP002151287
- SALIN H. ET AL: 'Sensitive and Quantitative Co-detection of Two mRNA Species by Double Radioactive In Situ Hybridization' THE JOURNAL OF HISTOCHEMISTRY & CYTOCHEMISTRY vol. 48, no. 12, December 2000, pages 1587 - 1591

## Description

### FIELD OF INVENTION

The present disclosure relates to compositions and methods for genetic analyses. More particularly, this disclosure provides compositions and methods for differential gene expression analyses. Even more particularly, the disclosure provides compositions and methods for analysing gene expression on biological material, such as tissue sections. This disclosure is based on hybridisation between the biological material and particular probes, more specifically in situ hybridisation with radioactive probes. This disclosure relates more preferably to differential gene expression analyses on biological material using particular probes with distinct radioactive labels. The present teaching can be used to detect or monitor gene expression or to quantitatively compare gene expression (e.g., differential gene expression screening), for instance, and is suitable for use in research, diagnostic and many pharmacogenomics applications, for instance.

### BACKGROUND

Various methods of genetic analysis or target nucleic acid detection have been described in the art, based on hybridisation with probes specific for the target gene (or nucleic acid). Such methods essentially comprise contacting a biological sample to be analysed with the probe, under conditions allowing nucleic acid hybridisation, and detecting the formation of hybrids, as an indication of the presence of the target nucleic acid in the sample. These techniques have been used to detect the presence of a nucleic acid, to monitor gene expression and/or regulation, to compare gene expression in different samples, etc.

In this regard, the *in situ* hybridisation method (ISH) is a common procedure for the detection of genetic material. It reaches a large number of biological fields such as anatomy, cellular biology and gene expression regulations. Since 1990, the characterisation of numerous genes and cDNAs but also the rapid development of molecular biology techniques has allowed the diffusion, refinement and user friendliness of ISH. It has become of great importance as a powerful method for localizing individual cells that contain particular species of mRNA within complex, heterogeneous tissues, such as the nervous system for instance. The basics of in situ hybridisation have been described for instance in "In situ hybridisation: a practical approach" (D.G. Wilkinson ed., Oxford University Press, 1992) or in Leitch et al. ("In situ hybridisation: a practical guide", Macroscopy handbooks 27, 1994). The anatomical data provided by ISH are very accurate and allows the performance regional, cellular and sub-cellular patterns of gene expressions. However, these prior art techniques of gene detection or analysis suffer from several drawbacks. In particular, to this date, fluorescent labelling has been used to allow multiparametric detection of genes, i.e., the simultaneous visualization of several genes. U-A-5 830 645 teaches the use of fluorochromes labels (in a context distinct from in situ hybridization). However, fluorescence does not allow quantification and is not sensitive enough to detect fine gene regulations or rare gene products. Furthermore, while quantitative data about the level of gene expression might be possible with the use of radioactive labelling, radioactive labelling has long been considered has unsuitable for frequent in situ hybridisation because of the technical difficulties inherent to radioactivity (security, length of acquisition, etc). Furthermore, such quantitative analyses were only achievable for one gene per experiment.

Therefore, it would be of major interest to gain the ability to routinely and precisely detect and quantify several mRNAs on the same tissue section and at a cellular level. It would be very advantageous to provide methods that would be sensitive, reliable, and allow detection and quantification of genes or gene products that are present or altered at low levels.

WO 97 40189 describes a method for the quantification and discovery of a target gene sequence in at least one or two physically distinct samples of cells that have been previously fixed, comprising exposing the fixed cells to a labelled nucleic acid probe. Labels such as radionuclides may be used. WO 98 38329 describes methods for identifying targets of a drug in a cell. However, these documents do not teach a simultaneous co-detection of different targets using different probes with distinct radiolabels.
US-A-5 928 870 discloses a method of detecting target nucleic acids in a biological sample, wherein a biological sample can be contacted with different sets of radioactive probes. However, this method does not allow simultaneous detection or measurement of the differently labelled nucleic acids. FR-A-2 772 484 and Laniece et al. ("A new high resolution for the quantitative analysis of radiolabelled molecules in tissue section" Journal of Neuroscience Methods, vol. 86, 1998, pages 1-5) describe a process to generate pictures of a substrate using radioactive labels. Pietu G et al. ["The Genexpress IMAGE knowledge base of the human brain transcriptome: a prototype integrated resource for functional and computational genomic's GENOME RESEARCH, vol. 9, no. 2, - February 1999 (1999-02) pages 195-209)] describes a kit comprising radioactive nucleotides.

### SUMMARY OF INVENTION

The disclosure provides methods and compositions for simultaneous detection and/or discrimination of target nucleic acids in a sample, using radioactive probes.

The disclosure provides methods and compositions for simultaneous visualization and/or quantification of several nucleic acids in a biological sample, using radioactive probes.

Several sets of radio-labelled probes that specifically hybridise with target nucleic acids and exhibit different (distinguishable) radiolabels are more specifically used.

Methods that allow co-detection and quantitative analysis of gene expression using radioactive probes are herein described. It is now possible to differentiate gene expression using radioactive probes on the same tissue sample, more particularly in the same cell.

The instant invention describes more specifically the simultaneous hybridisation and visualization of two radioactive probes on the same tissue section, each probe being labelled with different radio-elements (³³P/³⁵S/³H/³²P/¹²⁵I, etc.). Taking in consideration the specific disintegration activity difference between various radiolabelled nucleotides, the invention also discloses preferred methods and conditions allowing the use of these (five) different radioactive nucleotides to differently label different oligonucleotide probes that would be hybridised on the same tissue section and efficiently discriminate the probes on the same sample.

It is herein described a method of detecting target nucleic acids in a biological sample, said method comprising:
a) contacting the biological sample with at least two sets of radioactive probes, the probes of the first set being specific for a first target nucleic acid and labelled with a first radio-label, and the probes of the second set being specific for a second target nucleic acid and labelled with a second radio-label, and
b) detecting said first and second target nucleic acids in the biological sample by assessing the formation of hybrids between the probes and the sample.

In particular variants, the probes may be DNA molecules between 15 and 500 base-pairs-long, even more preferably single stranded DNA oligonucleotides between 15 and 500 bases long, or RNA molecules, between 15 and 3000 bases long.

In further particular variants, the probes may be labelled by (i) a 3' radioactive tracer, preferably a 3', 5-100 long, radiolabelled nucleic acid tail, (ii) a 5' radioactive tracer, and/or (iii) insertion in their sequence of radioactive nucleotides, i.e., comprise, in their sequence, radioactive nucleotides.

Particular ways of carrying out the instant invention comprise:
- using two sets of probes comprised of oligonucleotide probes,
- using two sets of probes comprising a 3' radioactive tracer or a 5' radioactive tracer or comprising, in their sequence, radioactive nucleotides, the probes of the two sets comprising more preferably a 3' radioactive tracer,
- using a first and second radioelements having a different emission-energy spectra, preferably a first set of probes labelled with tritium and a second set of probes labelled with a radioisotope selected from ³⁵S, ³³P, ³²P and ¹²⁵I.

According to specific embodiments, the two sets of probes are contacted simultaneously or sequentially with the biological sample, preferably using sets of probes having a similar specific disintegration activity, and using essentially similar amounts of each set of probes and/or the targeted nucleic acids are detected by assessing simultaneously the formation of hybrids between the two sets of probes and the sample.

According to other preferred aspects of the disclosure, the biological sample is a mammalian tissue sample, preferably a tissue section, and several biological samples are tested in parallel, preferably after being deposited on one or several supports, preferably glass support.

In this regard, another object of the disclosure resides in a method of simultaneously detecting target nucleic acids in several biological samples, comprising:
a) providing biological samples on one or several supports, preferably glass supports,
b) contacting, in parallel, the biological samples on the support(s) with at least two sets of radioactive probes, the probes of the first set being specific for a first target nucleic acid and labelled with a first radio-element, and the probes of the second set being specific for a second target nucleic acid and labelled with a second radio-element, and
c) simultaneously detecting said first and second target nucleic acids in the biological samples by assessing the formation of hybrids between the probes and the samples.

An other specific object of this disclosure is a method of detecting target nucleic acids in a biological sample, wherein each biological sample is contacted-, in parallel with the following at least two sets of probes:
a) probes of a first set specific for a first target nucleic acid and labelled with a first radio-label and probes of a second set specific for a second target nucleic acid and labelled with a second radio-label,
b) probes of the first set specific for the first target nucleic acid and labelled with the second radio-label and probes of the second set specific for the second target nucleic acid and labelled with the first radio-label,
and wherein the method further comprises assessing the formation of hybrids between the probes and the samples.

A method is further herein disclosed for comparing target gene expression in at least two biological samples, said method comprising:
a) contacting, in parallel, the biological samples (preferably on one or several supports) with at least two sets of radioactive probes, the probes of the first set being specific for a first target nucleic acid and labelled with a first radio-element, and the probes of the second set being specific for a second target nucleic acid and labelled with a second radio-element,
b) assessing the formation of hybrids between the probes and the samples, and
c) quantitatively comparing target gene expression in said samples by comparing the relative amount of hybrids formed between the samples.

Further aspects of the disclosure include the use of nucleic acid probes and especially the use of a RNA molecule or set of probes comprising radioactive nucleotides labelled with tritium, as well as an isolated nucleic acid molecule, wherein the molecule is single strand, comprises a 15-500 bases-long sequence, preferably 15-250, more preferably 15-100, which is complementary to a target nucleic acid, and comprises a 3' tritiated nucleotide tail, for in vitro or ex vivo gene expression analysis on a biological sample.

This disclosure also relates to the use of two radioactive probes with different nucleic acid sequences and different radioactive label, for in vitro or ex vivo gene expression analysis on a biological sample.

The probes may further be used in combination with other labelled probes or detection reagents or techniques, in order to provide further detailed information about a tissue sample. Such additional probes or reagents include fluorescent probes as well as labelled antibodies, specific for proteins or polypeptides. In this respect, the simultaneous detection target nucleic acids (using radiolabelled probe(s)) and target polypeptides (preferably using labelled antibodies, such as fluorescent-, enzymatic-, chemical- or radio-labelled antibodies) is herein disclosed.

In this regard, a particular variant herein disclosed resides in a method as defined above, further comprising contacting the biological sample(s) with a non-radioactive probe and/or an affinity reagent to detect additional target nucleic acid(s), polypeptide(s) or cellular component(s).

In a particular embodiment, a further detection reagent is a radiolabelled antibody, and the antibody is used in combination with a radiolabelled nucleic acid probe. More generally, this invention can be used for simultaneous detection or quantification of at least two target components of a cell or tissue (including nucleic acid, polypeptide, organelle) using two differently radiolabelled detection reagents (e.g., two nucleic acid probes, two antibodies, one nucleic acid probe and one antibody, etc.).

The disclosure also encompasses kits for nucleic acid (or other components) detection comprising radioactive nucleotides (or other detection reagents such as antibodies), enzymes and/or protocols for radioactive labelling of nucleic acid probes or antibodies as well as, more generally, any kit for implementing a method as defined above, comprising the reagents, supports and/or protocols for labelling, hybridisation and/or readout.

This invention can be used in many different technical areas, with virtually every type of biological material.

### DETAILED DESCRIPTION OF THE INVENTION

As indicated, the present invention resides in methods of detecting gene expression or regulation using particular probes. The present invention will now be disclosed in further details, the details being merely illustrative and not limiting the scope of the invention.

### The probe

The probe may be any nucleic acid molecule comprising a region of pre-determined sequence, more preferably any single-strand nucleic acid molecule comprising a region of pre-determined sequence. The region of pre-determined sequence comprises at least 15 consecutive nucleotides, more preferably at least 20 consecutive nucleotides. The sequence of this region is determined according to the target nucleic acid molecule which is to be detected or monitored in the biological sample. The target nucleic acid may be any (portion of a) gene, RNA, chromosome, viral genome, mitochondria, plasmid, episome, etc. For instance, where a particular gene or RNA is to be detected or monitored, the sequence of the region is complementary to said gene or RNA, preferably perfectly complementary, in order to allow specific hybridisation therewith. Although perfect matching (or complementarity) is preferred, it should be understood that mismatches may be tolerated, as long as the probe can specifically hybridise with the target nucleic acid under appropriate stringency conditions. The probes can be designed to avoid likely homology regions amongst members of a family of gene transcripts or, conversely, they can be targeted against a conserved, usually translated region in order to detect the same gene transcript in various species, for instance. The probe may also be designed to hybridise with all splicing variants of a gene or, on the contrary, with only one particular splicing form of a selected gene. Furthermore, one of the sets of probes may be specific for a control reference nucleic acid.

The probe may be a DNA molecule or an RNA molecule or a PNA molecule. In a particular embodiment, the probe is single- or double-strand DNA molecule between about 15 and about 2000 base(-pair) long, more preferably between 15-500. In a preferred embodiment, the probe is a single-strand DNA molecule, such as an oligonucleotide, comprising between 15 and 500 nucleotides, preferably between 15 and 100 nucleotides, more preferably between 20 and 50 nucleotides, even more preferably between 25 and 50 nucleotides. It should be understood that the size of the oligonucleotide probe may be adapted by the skilled person, and may be possibly larger than above indicated. Preferably, the size should allow specific hybridisation of the probe with a target nucleic acid, and thus include at least 10 or 15 nucleotides. The oligonucleotide may be produced according to conventional techniques, such as through DNA synthesizer, by any synthetic or semi-synthetic method, DNA cloning, digestion, ligation, and the like. Furthermore, the oligonucleotide may contain modified bases or may be further modified in order to increase its stability, or the stability of the hybrid, for instance. Such modifications include chemical modifications, enzymatic modifications, etc. In particular, the oligonucleotide probe may comprise modified nucleotides (e.g., biotinylated), modified bounds (phosphorothioates, etc.), intercalating agents (ethidium, etc.), etc.

The probe can also be a single-strand RNA molecule, comprising between 15 and 3000 nucleotides, more preferably between 20 and 2000 nucleotides, even more preferably between 25 and 1000 nucleotides. The RNA molecule may be produced according to conventional techniques, such as through RNA synthesizer or, preferably, by in vitro transcription from a DNA sequence encoding the same. This production method is preferred since it allows the production of large amounts of long (i.e., above 3000 nucleotides long) RNA probes. If needed, the RNA molecule may be further modified in order to increase its stability, or the stability of the hybrid, for instance. Such modifications include chemical modifications, enzymatic modifications, etc.

The probe can also be a molecule different than a nucleic acid, more precisely any kind of other molecule that has the ability to specifically bind (or interact with) the compounds to be detected in the sample. For example and without any limitation in the nature of the probe, the probe may be an immunoglobulin (antibody) or a mix of different immunoglobulins, or the ligand of a given receptor-protein, or an antigen that will bind immunoglobulin or immunoglobulin-like proteins in the sample, etc. It should be understood that the present invention is based on the concept of simultaneous detection of different biological compounds in a sample with differently-labelled radioactive probes, and is not limited to the simultaneous detection of nucleic acids. Different compounds of a different nature in a same sample may thus also be simultaneously detected with the use of probes of different natures.

### Labelling

As indicated above, this invention resides in the use of radioactive probes, more specifically probes having distinct radioactive labels, in order to detect and monitor fine gene expression and regulation within biological samples. More specifically, the invention resides in the use of at least two sets of probes having a different radioactive label, the probes of the first set being specific for a first target nucleic acid and the probes of the second set being specific for a second target nucleic acid.

### Radiolabel

As indicated, the invention uses at least two sets of probes which are differently radiolabelled. Preferably, each set of probes contains probes labelled with one particular radioelement, which can be distinguished from the radioelement used for the other set(s) of probes.

In this regard, many radio-elements or isotopes can be used for the labelling of the probes. Specific examples of isotopes include ³H, ³⁵S, ³³P, ³²P, ¹⁴C, ¹²⁵I, and the like.

Preferably, the invention uses at least two sets of probes as defined above, the sets being labelled with radioelements having a different emission energy, more preferably a distinguishable emission energy spectra. More preferably, the mean emission energy of the radioelements used should differ of at least 10 Kev, more preferably at least 20 Kev, even more preferably at least 30 Kev. Table 1 below discloses the emission energy, resolution and period for the preferred radioelements to be used in this invention.

**TABLE 1**

| Radioisotopes | emission | mean energy (KeV) | max. energy (KeV) | resolution (µm) | Period |
|---|---|---|---|---|---|
| ³H | β⁻ | 5.7 | 18.6 | 0.5-5 | 12.3 years |
| ¹⁴C | β⁻ | 49.4 | 156.5 | 10-20 | 5730 years |
| ³⁵S | β⁻ | 48.8 | 167.5 | 10-15 | 87.4 days |
| ³³P | β⁻ | 76.4 | 248.5 | 15-20 | 25.6 days |
| ³²P | β⁻ | 695.5 | 1710.4 | 20-30 | 14.3 days |
| ¹²⁵I | e⁻ auger | 3.7 (79.3) | | 1-10 | 59.9 days |
| | | 22.7 (19.9%) | | | |
| | | 30.6 (10.7%) | | | |
| | | 34.5 (3.3%) | | | |
| | γ | 35.5 (6.7%) | | | |
| | X | 27.2 (39.6%) | | | |
| | | 27.4 (73.8%) | | | |
| | | 30.9 (21.3%) | | | |
| | | 31.7 (4.3%) | | | |

Table 1 shows that ³H emission energy spectrum is clearly distinguishable from that of ³⁵S, ³³P and ³²P, for instance. In a preferred embodiment, one set of probes is thus labelled with tritium and another set of probes is labelled with a radioisotope selected from ³⁵S, ³³P and ³²P. The examples disclosed below provide evidence that such sets of differently labelled probes can be used efficiently to simultaneously detect and discriminate target nucleic acids in a same biological sample, with a very high sensitivity.

Radioactive nucleotides to be used in this invention include natural and non-natural radiolabelled nucleotides, more preferably radiolabelled nucleotides selected from ATP, dATP, CTP, dCTP, GTP, dGTP, UTP, dUTP, TTP, dTTP. Such nucleotides are commercially available, or may be produced by conventional chemical methods. More preferred radiolabelled nucleotides to be used in the instant invention are listed in Table 2 below:

**TABLE 2**

| Isotope | Nucleotide | ref. | specif. Activity Ci/mmole |
|---|---|---|---|
| | dATP | TRK 633 | 50-100 |
| | | TRK 347 | 1-10 |
| ³H | dCTP | TRK 625 | 50-85 |
| | | TRK 352 | 15-30 |
| | dGTP | TRK 627 | 25-50 |
| | | TRK 350 | 5-20 |
| | dUTP | TRK 351 | 5-30 |
| | d ATPαS | SJ 1304, 304,264, 1334, 1300 | 400-1000 |
| ³⁵S | d CTPαS | SJ 1305, 305, 1302 | 400-1000 |
| | UTPαS | SJ 1303, 603, 263 | 400-1000 |
| | (γ) ATP | BF1000 | ≥ 2500 |
| | (α) dATP | BF1001 | ≥ 2500 |
| ³³P | (α) dCTP | BF1003 | ≥ 2500 |
| | (α) CTP | BF1012 | ≥ 2500 |
| | (α) UTP | BF1002 | ≥ 2500 |
| | (α) dATP | PB10474, 10204, 10384, 10164 | 400-6000 |
| | (α) ATP | PB10200, 10160 | 400-3000 |
| | (γ) ATP | PB218, 168, 10218, 10168 | 3000-5000 |
| | (α) ddATP | PB10235, 10233 | 3000 - > 5000 |
| | (α) dCTP | PB10475, 10205, 10385, 10165 | 400-6000 |
| ³²P | (α) CTP | PB10202, 20382, 10162, 40382 | 400-3000 |
| | (α) dGTP | PB10206, 10386, 10166 | 400-3000 |
| | (α) GTP | PB10201, 10161 | 400-3000 |
| | (γ) GTP | PB 10244 | > 5000 |
| | (α) dTTP | PB10207, 10387, 10167 | 400-3000 |
| | (α) UTP | PB10163, 10203, 20383 | 400-3000 |
| ¹²⁵I | dCTP | NEX 074 | 2200 |

Even more preferably, radiolabelled nucleotides with high specific disintegration activity are being used, in order to produce probes with high specific disintegration activity value, as will be further disclosed below.

The probes may be radio-labelled according to different techniques.

### Post-synthesis labelling

In a first embodiment, the probes are labelled post-synthesis. In this embodiment, the probes are first produced and then labelled, using a selected radio-isotope.

Post-synthesis labelling may be performed according to various strategies. In the preferred variant of this invention, the probes are labelled by addition of a terminal radioactive tracer to the probes. In a more preferred embodiment, the terminal radioactive tracer comprises one or several radioactive nucleotides having the same radio-isotope, i.e., a radioactive tail. The tail may be a homopolymer, i.e., composed of the same repeated nucleotide, or a heteropolymer, i.e., composed of several different nucleotides. Where a heteropolymer tail is used, the sequence should preferably be determined so as not to interfere with the hybridisation of the probe and not to form secondary structures (loops, etc.).

In a preferred embodiment, the terminal radioactive tracer is a homopolymer tail, more preferably a 3' (homopolymer)-tail.

Furthermore, in the tail, all or only a part of the nucleotides may be radio-labelled. Indeed, by adapting the concentration or proportion of radioactive nucleotides in the tail, it is possible to control or adjust the specific disintegration activity of the probe. Obviously, the radioactive nucleotides present in the tail should preferably all bear the same radio-isotope so that each set of probes is characterized by a particular radioisotope.

The specific disintegration activity of the probes may be further adapted by controlling or adjusting the length of the tail. In this regard, in a particular embodiment of this invention, the tail comprises preferably 5 to 100 nucleotides, more preferably between 5 and 50 nucleotides, even more preferably between 5 and 30 nucleotides, and even more preferably at least 25% of the nucleotides in the tail are radiolabelled.

The tail may be produced either separately and then linked to the probe, or by direct sequential addition of the nucleotides to the probe.

In this regard, in a preferred embodiment, the probe is labelled by contacting the probe with radioactive nucleotides in the presence of an enzyme that catalyses the 3' binding of nucleotides. A typical enzyme to be used is a terminal transferase. As indicated above, the concentration of the nucleotides and the proportion of radioactive and non radioactive nucleotides may be adapted to adjust the specific disintegration activity of the probe.

In a preferred variant, the probe comprises a 3'-tail produced by sequential addition to the probe of 5-100 nucleotides, all or part of which bearing a selected radiolabel. More preferably, the 3' tail is a 5-100 bases long homopolymer, preferably a polyA, polyC, polyG, polyT or polyU tail, in which all or part of the nucleotides bear a selected radioisotope.

Post synthesis labelling may also be performed by addition of radiolabelled phosphates (e.g., (γATP, γGTP)³²P, γATP³³P, ³⁵S-thio-phosphates) to the 5' end of the probes, using suitable enzymes such as T4 kinase. Such method may be used alone or in combination with others, since it may not allow very high specific disintegration activity to be achieved.

### Labelling during synthesis

The probes can also be labelled during their synthesis. In this embodiment, radiolabelled nucleotides are incorporated into the probe during the synthesis. This embodiment is particularly suited for RNA probes which are produced in in vitro transcription systems as mentioned above. As for post-synthesis labelling, the specific disintegration activity of the probe can be adjusted by controlling the concentration of radiolabelled nucleotide in the synthesis medium.

In a preferred embodiment, each set of probes to be used in the same assay should be labelled using the same technique (i.e., post-synthesis or during synthesis, 3' tail vs 5' phosphate, etc). Even more preferably, to perform the present invention, the probes of each set contain a 3'-tail, more preferably a 3'-homopolymer tail, even more preferably a 3'-homopolymer tail comprising between about 15 and about 85 nucleotides.

### Non-radioactive probes or labelling

While the invention discloses methods of detecting (or quantifying or visualizing) nucleic acids in samples using at least two differently radiolabelled sets of probes, it should be understood that the invention may be performed by combining said radiolabelled probes with any other probe or detection reagent, in order to obtain a further detailed image of the sample.

In this regard, additional non-radioactive probes may be used, such as fluorescent probes, in combination with the above radioactive probes, so that additional genes or RNAs can be monitored simultaneously in the sample, or to introduce additional controls.

Also, additional detection reagents, such as affinity reagents, may be used in order to further detect proteins (or polypeptides), receptors, organelles, etc. within the sample. Such reagents include immunomolecules such as antibodies (or fragments or derivatives thereof), which can be labelled according to conventional techniques (enzymatic, fluorescent, chemical, etc.).

### The biological sample

The biological sample may be any mammalian biological material such as tissue sample, organ sample, biopsy, skin sample, biological fluid, bone marrow, nervous tissue (e.g., brain tissue), etc. The biological material may also comprise plant tissue or cells, prokaryotic cells, lower eukaryotic cells, established cell cultures, viruses, any other unicellular organism, etc. Because of the high sensibility and high reproducibility of the present method, very low quantities of biological material may be used, and the invention can be applied to essentially all types of biological material. The invention is particularly suited for detecting rare mRNA species as well as fine gene expression regulation within complex tissues, such as nervous tissue.

The biological sample of a mammalian or plant tissue is typically prepared by cutting fresh-frozen tissues on a cryostat, for example 10-15 µm thick sections.

Alternatively, the biological sample may be prepared from any tissue by fixation in suitable substances such as paraffin. The tissue may then be cut in a vibratome to produce appropriate section.

The biological material is preferably deposited on a support prior to the contacting with the probes. The support may be any suitable support for genetic analysis, including plastic, nylon, glass, silicium, etc. A typical example of glass slide includes the SuperFrost^{R} Plus (Menzel-Glaser, Germany). Preferably, the support comprises glass, such as glass slides. The support may be pre-treated to ensure adhesion or immobilization of the biological sample thereto (e.g., gelatine-coated). The biological sample (or the support) may then be stored for later analysis, or use directly. Where storage is performed, freezing may be used, such as freezing at -20°C or -80°C, for instance, preferably after air drying.

In a preferred variant of this invention, several biological samples are tested in parallel. The various samples may be deposited on the same support, or on separate supports. In a preferred embodiment, several samples are deposited on the same support. The samples may be different sections of a same tissue, a tissue sample or cell population at various stages (maturation, treatment with a compound, apoptotic, cancerous, etc.); different samples of the same tissue or cell population from different origins (e.g., different subjects, different species, etc.). As indicated before, it is believed that the instant invention can be used with essentially any biological sample and should not be limited to particular applications. Preferably, the sample is a mammalian tissue sample, such as nervous cells, blood cells, tumor cells, embryonic cells, etc. It can be, for instance, a human tissue sample or a rodent tissue sample.

Prior to contacting the biological material(s) with the probes, the biological material(s) may be subjected to various pretreatments, such as fixation, permeabilization, delipidation, etc. In a preferred embodiment, the sample is subjected to fixation, using conventional agents. Fixation allows to maintain the sample in its status (e.g., to avoid RNA degradation, protease activity, nuclease activity, etc.). Preferably, the sample is fixed using formaldehyde, paraformaldehyde (PFA), glutaraldehyde, Bouin solution, etc.. More preferably, the samples are subjected to fixation in the presence of a PFA solution (e.g., 4%). For samples that are not frozen (e.g., in paraffin), they may be subjected to fixation prior to their deposit on the support.

While additional pretreatments may be performed, the invention can be used efficiently with no need for further treatments such as permeabilization, especially with frozen samples. This represents another advantage of the instant invention. Where samples in paraffin are used, they are preferably treated with protease to increase permeability.

### Hybridisation

The present invention now provides, for the first time, evidence that differently labelled sets of radioactive probes can be used simultaneously on a biological sample and that the signals emitted can be discriminated. The invention demonstrates that the discrimination can be made by adapting the specific disintegration activity of the probes and controlling the hybridisation conditions, as will be discussed below.

In the present invention, the sample is contacted with at least two sets of probes as defined above. The contacting allows formation of hybrids between the nucleic acids of the sample and the probes, where target nucleic acid is present in the sample. Accordingly, the contacting shall be made under conditions sufficient to allow nucleic acid hybridisation to occur. Conditions for forming hybridisation have been disclosed for instant in Maniatis et al (Molecular Cloning, a Laboratory Manual, 1989) or in Nucleic Acid Hybridization, A practical approach IRL Press, Wash. DC (1985).

In this regard, in order to ensure high sensitivity of the method, the contacting step is preferably performed under conditions allowing the probes to hybridise with the target nucleic acid as well as, potentially, with non-target (i.e., aspecific) nucleic acids, non-specific hybridisation being eliminated or reduced by suitable washing conditions. The hybridisation condition can be adjusted by the skilled artisan. Essentially, hybridisation can be controlled by the hybridisation medium and temperature. In this respect, hybridisation is preferably performed at temperatures between about 30 and about 70 °C (high temperatures 60-70°C being preferred for RNA probes). Furthermore, the hybridisation medium generally comprises standard saline citrate solution (SSC) at moderate saline strength. Specific hybridisation conditions are disclosed in the examples and can be adapted by the skilled person. Typically, the hybridisation medium comprises Denhardt's solution and SSC solution. Furthermore, the hybridisation medium may comprise additional agents that reduce non-specific signal or probes rearrangements, for instance. In this respect, the hybridisation medium generally comprises dithiothreitol (DTT) and/or formamide. In addition, in a particular aspect of this invention, hybridisation is performed in the presence of competitor nucleic acid, to reduce background signal. In particular, where the probes contain a labelled nucleotide tail, the contacting step can be performed in the presence of un-labelled oligonucleotides complementary to the tail. The competitor nucleic acid may be used simultaneously with the probes, or contacted with the sample prior to the probe.

A preferred hybridisation medium thus comprises SSC, DDT, formamide and a competitor nucleic acid.

In a typical experiment, each biological sample is contacted with a hybridisation medium in the presence of at least two radioactive sets of probes, for a period of time sufficient to ensure formation of hybrids, for instance between 1 hour to 12 hours.

In order to allow efficient discrimination and visualization of each set of probes (i.e., each target nucleic acid) on the sample(s), it is preferred to use particular amounts of sets of probes, with a particular specific disintegration activity, for the hybridisation step. In this regard, the invention now demonstrates that efficient discrimination and quantification of the different labels is best achieved where both sets of probes have a specific disintegration activity comprised between about 5.10⁷ and 5.10¹⁰ cpm/µg, more preferably between about 10⁸ and 10¹⁰ cpm/µg, even more preferably between about 5.10⁸ and 5.10⁹ cpm/µg. A more preferred way of performing the invention comprises the use of two sets of probes having essentially the same specific disintegration activity, i.e., not differing by more than about 3 times from each other(s), more preferably not by more than about two times. The specific disintegration activity of the probes can be adjusted by the choice of the nucleotide (see table 2 above) and the conditions of the labelling method, as discussed above. In this respect, where the selected radionucleotides have a distinct specific disintegration activity, the labelling conditions should be adjusted to ensure that the labelled probes have essentially a similar specific disintegration activity.

In addition, in performing the hybridisation, it is also recommended to use similar amounts of each set of probes, so that more reliable and comparable results are obtained. In this regard, when the probes are nucleic acid molecules, typical experiments are performed using between 0.05 and 0.5 pmoles of probes of each set per each 1 cm² surface of the biological sample to be investigated (for example tissue slice), more preferably between 0.05 and 0.2 pmole per each 1 cm². While these are preferred conditions allowing discrimination of nucleic acids present at very broad spectrum of levels (i.e., from rare to very abundant) and from virtually any type of biological material, it should be understood that the molarity (or amount) of probes of each set can be adjusted by the skilled artisan to the specific conditions or biological samples.

The present invention can be implemented using a variety of nucleic acid probes, as described above. These probes may vary in length as well as in nature. In this regard, it is possible to use, in performing the invention, two nucleic acid probes of the same or different nature. More particularly, the nucleic acid probes may be either both oligonucleotides, DNAs, RNAs, PNAs, etc. (i.e., of the same nature) or of a different nature, e.g., oligonucleotide probes and DNA probes, oligonucleotide probes and RNA probes, DNA probes and RNA probes, etc. Generally, any probe mixture or combination can be used in the present invention.

In order to perform simultaneous (in situ) hybridisation of differently radiolabelled probes, each labelled set of probes may be contacted simultaneously with the sample. However, it should be understood that the term "simultaneously" indicates that the readout of the results concerning the two sets of probes (or more) should be performed at the same time, whatever the sequence in which the sets of probes are contacted with the sample. In some cases, the hybridisation may be performed with the two sets of probes essentially at the same time, so that only one hybridisation/washing round is performed (for instance when the two sets of probes are both nucleic acid probes with a similar ability to hybridise to their target molecule), but "simultaneous" does not require that the sets of probes be contacted with the sample at exactly the same time. In other cases, the two sets of probes may be contacted sequentially with the sample (one after another and in separate steps). As an example, such sequential procedures may be used when the two sets of probes are of a different chemical nature (such as an antibody and a nucleic acid sequence, etc.) or when the compounds to be detected in the sample are of different organic natures (such as a messenger RNA and a protein, etc.).

In a particular embodiment, the sets of probes are mixed with the hybridisation medium, and the samples are then exposed to the resulting solution.

In another embodiment, the samples are first exposed to the hybridisation medium, and the sets of probes are then added, either simultaneously or sequentially.

Typically, when the probes are nucleic acid molecules, between 20 to 200 µl of hybridisation medium is added to each sample for each 4 cm² surface of the sample, or for one whole standard-sized microscope glass slide. The exposure time may vary, for instance, from 1 or several hours to one or several days. Preferably, the hybridisation lasts for less than about 24 hours, typically between 1 and 12 hours.

The samples are then rinsed to eliminate unbound probes as well as non-specific hybridisation. In this regard, any conventional washing solution may be used, such as saline solutions. Preferably, the samples are washed using saline citrate solution (SSC) comprising DTT, in order to eliminate non-specific hybrids formed. Preferred washing conditions use DTT (e.g., 10 mM) at elevated temperatures, typically 10-20°C below the theoretical melting temperature, preferably above 40°C, more preferably above 45°C, in a specific example above about 50°C. Several washings may be performed to increase the selectivity of the method.

The samples are then preferably dried (e.g., dehydrated) and apposed to scintillating paper for subsequent measure of the radioactivity (readout).

### Readout

In order to assess hybrid formation on the samples and to detect the presence or amount of target nucleic acids in said samples, the method comprises (i) washing the unbound probe (as described above) and (ii) detecting radioactivity (i.e., the first and second radiolabel) on the sample.

Radioactivity detection and discrimination may be achieved by different techniques using quantitative imaging devices such as Beta Imager (50-250 µm depending on the radioisotope used) and the Micro Imager that provides direct detection by the solid scintillator sheet principle and allows cellular size expression analysis (15 µm).

Preferably, acquisition of radioactive images is performed with a Micro Imager (Biospace Mesures, Paris, France), a real time, high-resolution digital autoradiography system. The instrument allows precise quantitative imaging of tissue section with a spatial resolution of 15 µm and a pixel size of 5 µm. Imaging is performed by optical contact between the radiolabeled sample, a thin foil of scintillating paper, and an intensified CCD camera. Beta particles are identified through light spot emission in the scintillating foil, allowing thus filtering of the background noise as well as filtering of emissions due to isotopes of different energies (FR2,772,484). The instrument is particularly well suited to the imaging and quantification of dual labelled samples and in particular to the simultaneous measurement of differential gene expression.

As an example, when the currently available Micro Imager device is used, imaging is performed on a 24 mm x 32-mm area. An automated sample feeder allows successive imaging of up to four slides. Detection threshold is kept to the very low level of 0,4 counts per minute per square millimetre for tritium labelling, and ten times lower for higher energy isotopes, a figure obtained thanks to the intrinsic noise suppression of the instrument. Because of the direct particle counting principle of the instrument, quantification is obtained with a precision better than 5%, without underexposure or saturation effects over four decades. Very fine variations of gene expression levels can therefore be measured with high accuracy. It should be understood that these parameters are only indicative and that larger areas of imaging do not fall beyond the scope of this invention.

In a preferred embodiment, radioactivity detection is thus performed by optical contact between the labelled sample, a thin foil of scintillating paper and an intensified CCD camera.

The invention can be used to detect gene expression in any biological sample, for research, diagnostic or any other experimental or industrial applications (pharmacogenomics, etc.). Gene expression may be used to identify a dysfunction, compare gene regulation, identify therapeutic genes, assess responsiveness of a subject, assess the presence of pathogenic agents (e.g., virus, bacteria, etc.) in a sample, etc.

Other aspects and advantages of the present invention will be described in the following examples, which should be regarded as illustrative and not limiting the scope of protection.

### LEGEND TO THE FIGURES

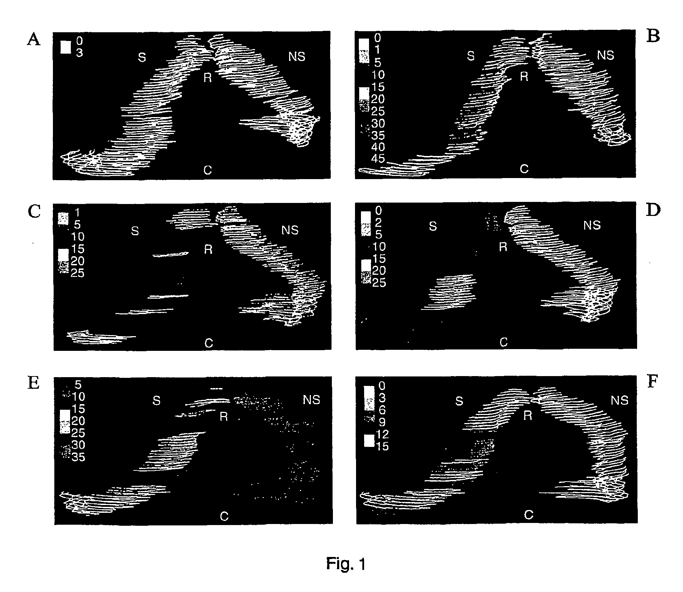

Figure 1: Three-dimensional reconstruction of the distribution of the mRNA of the three genes in the rat dentate gyrus following LTP induction. The expression of Homer mRNA in a control rat (panel A) and 3 hours (panel D) and 5 hours (panel F) after the induction of LTP is shown. The pattern of expression of Zif268 mRNA 30 minutes after the induction of LTP is shown in panel B, and the expression of syntaxin 1B is shown 3 hours (panel C) and 5 hours (panel E) following the induction of LTP. Note the heterogeneous level of expression of these genes in the stimulated side (S) of the dentate gyrus following the induction of LTP and the homogeneous pattern of expression of each of the genes in the non-stimulated (NS) side. The dentate gyrus is orientated from top to bottom in a rostro - caudal manner. mRNA abundance is expressed as mean optical density per pixel (ODp) according to the scale shown on the left of each panel.

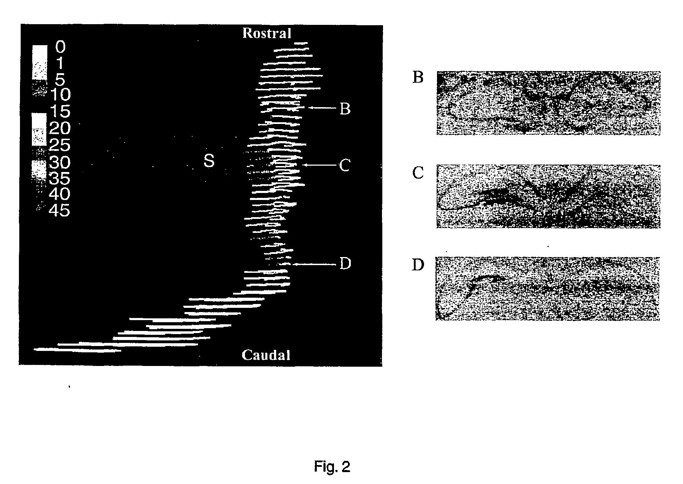

Figure 2. Heterogeneous spatial profile of Zif268 mRNA expression along the rostro-caudal axis of the stimulated side (S) of the dentate gyrus 30 minutes after LTP induction. In the three-dimensional reconstruction of the distribution of Zif268 mRNA (panel A), the letters B, C and D refer to the positions along the rostro-caudal axis that correspond to the autoradiographs of coronal sections in figures B, C and D, respectively. There is very little Zif268 mRNA on the non-stimulated side of the dentate gyrus. On the stimulated side, Zif268 is very weakly expressed in the rostral part (B); in the anterior part of the medial dentate gyrus, there is more Zif268 mRNA in the lower blade of the dentate gyrus (C), whereas in the posterior part, there is more in the upper blade (D). mRNA levels are expressed as mean optical density per pixel (B, C and D) according to the scale on the left of panel A.

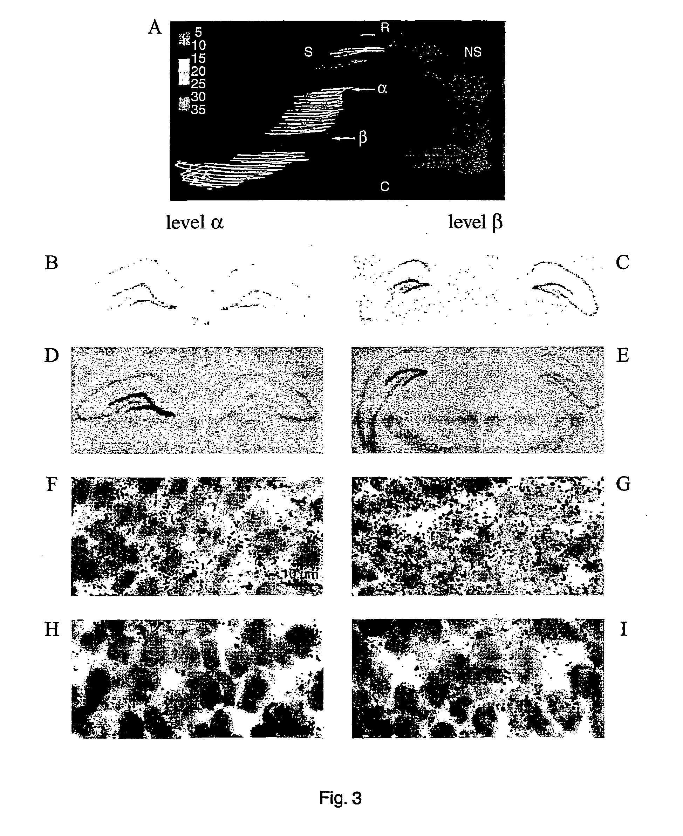

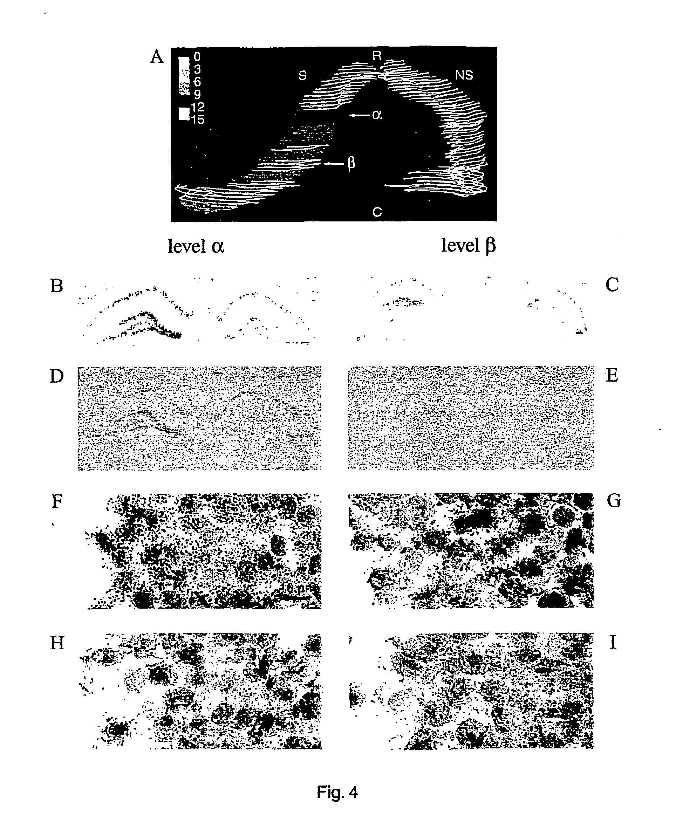

Figure 3: Spatial profile of the distribution of syntaxin 1B mRNA along the rostro-caudal axis of the dentate gyrus 5 hours after LTP induction. Panel A shows a 3-D reconstruction of the distribution of syntaxin 1B mRNA evidencing heterogeneous expression of the gene on the stimulated side (S). The rostro-caudal axis of the dentate gyrus is orientated from top to bottom. Figures B, D, F and H correspond to the α level of the dentate gyrus in panel A, and figures C, E, G and I correspond to the β level in panel A. (B and C) Coronal sections were simultaneously hybridised with the ³H-labelled probe for syntaxin 1B and the ³⁵S-labelled probe for Homer. Figures B and C correspond to ³H Beta disintegrations of the syntaxin 1B probe, and ³⁵S Beta disintegrations of the Homer probe correspond to panels B and C in figure 4. Figures D and E show coronal sections hybridised with only the ³⁵S-labelled syntaxin 1B probe. Figures F-I show light field microphotographs of emulsion dipped sections probed for syntaxin 1B alone, counterstained with Nissl, and correspond to the autoradiographs D and E. There is a greater abundance of silver grains at the β level of the dentate gyrus (panel G) than at the α level (panel F) on the stimulated side. In contrast, there are few silver grains in both the α (panel H) and β (panel I) levels of the non stimulated (NS) dentate gyrus.
Figure 4: Homer mRNA distribution along the rostro-caudal axis of the dentate gyrus 5 hours after LTP induction. Panel A is a 3-D reconstruction of the distribution of Homer mRNA, and shows the heterogeneous expression of the gene on the stimulated side (S). The rostro-caudal axis of the dentate gyrus is orientated from top to bottom. Five hours after LTP induction, the expression of Homer was differentially modulated along the rostro-caudal axis of the stimulated side (S) of the dentate gyrus. No changes were observed in the non-stimulated side (NS). Figures B, D, F and H correspond to the α level in panel A, and figures C, E, G and I to the β level. (B and C) Coronal sections were simultaneously hybridised with the ³H-labelled probe for syntaxin 1B and the ³⁵S-labelled for Homer. Figures B and C correspond to ³⁵S Beta disintegrations for Homer. The images of ³H Beta disintegrations correspond to the panels B and C of figure 3. Coronal sections D and E were hybridised with only the ³⁵S-labeled probe for Homer. Figures F-I are light field microphotographs of emulsion dipped sections counterstained with Nissl, and correspond to the sections in panels D and E. There is a greater abundance of silver grains at the α level of the dentate gyrus (panel F) than at the β level (panel G) on the stimulated side. Few silver grains are observed at the α (panel H) and β (panel I) levels of the non-stimulated (NS) dentate gyrus.

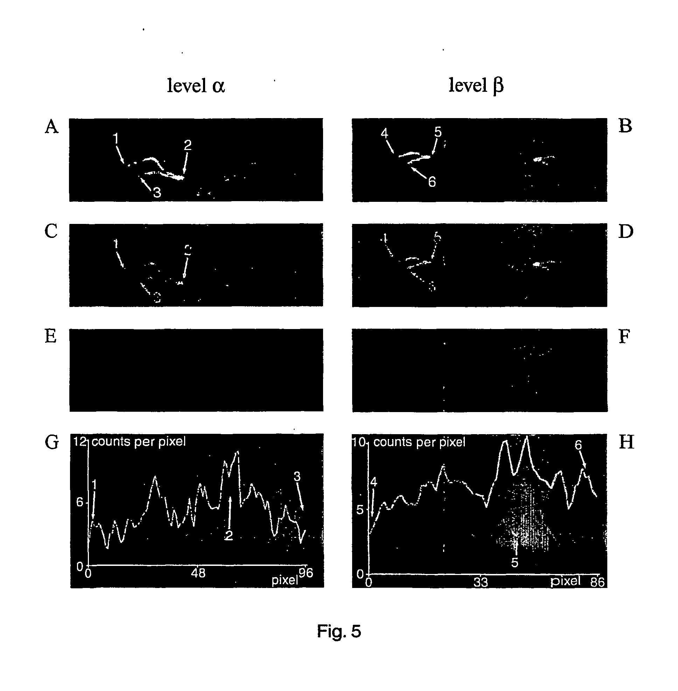

Figure 5: Coronal sections from a brain in which LTP was monitored for 5 hours, were simultaneously hybridised with the ³H-labelled probe for syntaxin 1B and the ³⁵S-labelled probe for Homer. Figures A, C and E correspond to the α level, and figures B, D and F to the β level of the dentate gyrus, shown in figures 3A and 4A. After digital acquisition of the radioactive images, the data were filtered to segregate the image corresponding to the ³H Beta disintegrations of the syntaxin 1B probe (panels C and D), from that corresponding to the ³⁵S Beta disintegrations of the Homer probe (panels E and F). Panels A and B represent double labelled images. The ³H-labelling for syntaxin 1B is represented in green, and the ³⁵S-labelling for Homer is represented in red. Where there is overlap in the expression of the two genes, the labelling is represented in shades of yellow on panels A and B. The graph in G corresponds to the quantification of each label in each pixel along the granule cell layer of the dentate gyrus at the α level: green and red correspond to syntaxin 1B and Homer, respectively. The numbered arrows in G correspond to the same numbers on the sections in panels A, C and E.

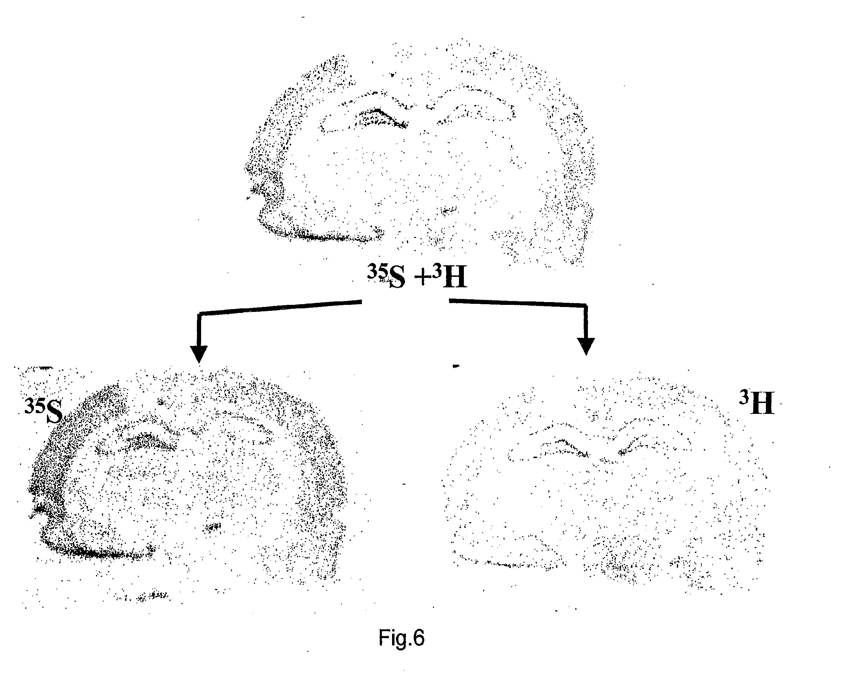

Figure 6: Detection and discrimination of radiolabelled probes on coronal sections.

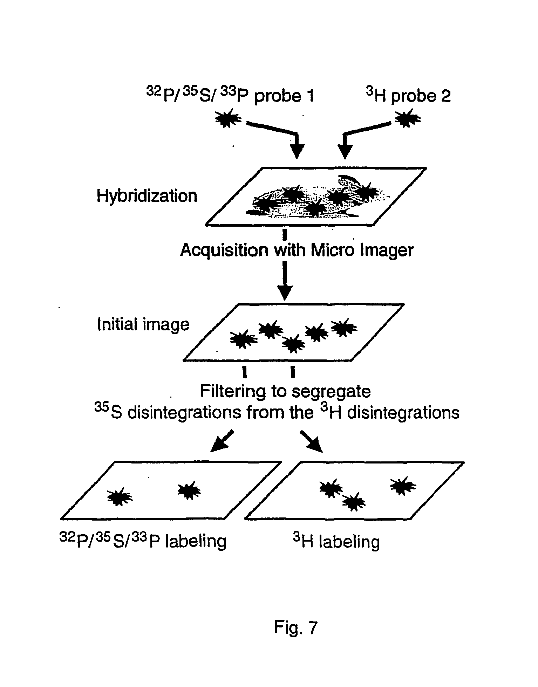

Figure 7: Principle of the double labelling technique in *in situ* hybridisation. Two differently labelled probes are simultaneously hybridised on a same tissue section. After washing, the section is read by the Micro Imager. The initial image acquired is then filtered to segregate the image corresponding to ³H Beta disintegrations from that corresponding to ³²P/³⁵S/³³P Beta disintegrations.

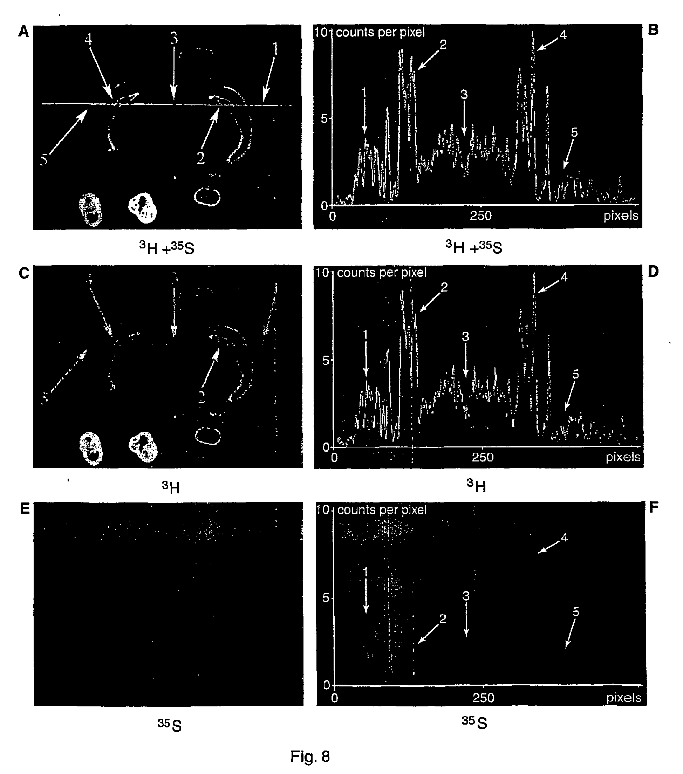

Figure 8: Visualisation of the results of a double radioactive *in situ* hybridisation. (A) Simultaneous visualisation of the both ³H- and ³⁵S-labelling. The ³H-labelling is here represented in green, the ³⁵S-labelling in red and the overlapping of the both labelling in yellow. (C) Visualisation of only ³H- labelling. (E) Visualisation of only ³⁵S-labelling. Below the three brain sections, a spot of ³H-labelled probe, one of a mix of ³H- and ³⁵S-labelled probes and another of ³⁵S-labelled probe were set down on the slides as controls for filtering allowing segregation of ³⁵S-beta from ³H-beta disintegrations. (B, D and F) Graphs corresponding to the respective contributions of each label to each pixel along the line drawn on the brain images. The green and red profiles correspond to the ³H-labelled probe and the ³⁵S-labelled probe together (B) or separately (D and F). The 5 arrows show 5 areas analysed in panels A, C and E, and the corresponding intensities of expression of the hybridised probes (B, D and F).

### EXAMPLES

### Example 1

### Tissue preparation

Coronal sections were cut at 15-20 µm in the dorsal part of rat dentate gyrus at -20°C on a Leitz cryostat. Sections were mounted onto superfrost treated slides, air dried and stored at -80°C until required. Frozen sections were first warmed to room temperature and then fixed in 4% paraformaldehyde in phosphate buffered saline (PBS, pH 7.4) for 20 min at room temperature, washed in PBS for 3 times (5 min), dried in absolute ethanol.

### DNA probe preparation

Antisense oligonucleotides were synthesized in-house on a Beckman Oligo 1000DNA synthesizer. Oligonucleotide sequences were designed complementary to rat mRNA-derived sequences available in published databases. The used probes were oligonucleotides complementary to specific regions of syntaxin 1B sequence (35-mer oligonucleotide sequence: 5'-GAT GTG TGG GGA GGG TCC TGG GGA AGA GAA GGG TA-3') and Homer sequence (39-mer oligonucleotide sequence: 5'-GGT CAG TTC CAT CTT CTC CTG CGA CTT CTC CTT TGC CAG-3'). Probes were 3'end-labelled with α-³⁵S-deoxyadenosine triphosphate (³⁵S-dATP, SJ 1334, Amersham) or deoxy[1',2',5,³H]cytidine 5' triphosphate (³H-dCTP,TRK.625, Amersham) in a tailing reaction, using terminal deoxynucleotide transferase (Amersham). 60 ng of each oligonucleotide was incubated in 40 µl buffer solution containing 8 µl of terminal transferase buffer x5 (M189A, Promega), 4 µl of ³⁵S-αdATP or 40 µl of ³H-dCTP (previously dehydrated and dissolved in 4 µl of distillated water) and 2 µl of terminal deoxynucleotide transferase ( E2230Z, Amersham). Purification of the labelled probes was performed on P10 column (150-4140, Biorad). The specific disintegration activity of each labelled probe was between 4x10⁸ and 9x10⁸ cpm/µg.

### In situ hybridisation

Sections were post-fixed in 4% paraformaldehyde in phosphate-buffered saline (PBS) immediately before hybridisation. The hybridisation solution was composed for 1 ml of :

| | |
|---|---|
| 500 µl deionized formamide | (50%)_{f} |
| 20 µl Denhart 50 | (X1)_{f} |
| 200 µl SSC X20 | (X4)_{f} |
| 100 µl DTT 1M | (100 mM)_{f} |
| 100 mg Dextran sulfate | (10%)_{f} |
| 25 µl yeast tRNA 10mg/ml | (250 mg/ml)_{f} |
| 25 µl poly A 10 mg/ml | (250 mg/ml)_{f} |
| 25 µl herring sperm DNA 10 mg/ml | (250 mg/ml)_{f} |

The two probes were simultaneously diluted to 1/100 with the hybridisation solution and 70 µl of the mixture were applied to each rat brain slice. Sections were incubated overnight at 50°C under parafilm Fuji, then washed twice for 15 min in 1X standard saline citrate (SSC)/10mM DTT at 53°C, twice for 15 min in 0.5X SSC/10mM DTT at 53°C and once in 0.5X SCC/10mM DTT at room temperature before being dried by dipping into an ethanol bath. Control experiments were performed either by displacing specific mRNA hybridisation by a 50-fold excess of unlabelled oligonucleotides or by using a sense oligonucleotide that yielded no signal in tissue sections.

### Double labelling in in situ hybridisation.

Acquisition of radioactive images was performed with a Micro Imager (Biospace Mesures, Paris, France) for 15 hours. The whole dentate gyrus of the brain slice was delimited and the number of disintegrations per area of this region was measured using β-Vision software (Biospace). The results are presented on Figure 6 and clearly demonstrate simultaneous visualization of both target nucleic acids in the tissue sample.

### Example 2

A dual detection method was performed essentially as described in Example 1, using the following hybridisation solution: hybridisation solution (Amersham, UK) supplemented with 40% v/v deionised formamide (MERCK), 50 µg/ml poly A⁺ (Sigma) and 50 mM 4-dithriothreitol (DDT, Euromedex).

As a result, the two target nucleic acids of the samples were clearly detected and discriminated from each other on the same samples.

### Example 3

A dual detection method was performed essentially as described in Example 1, except that one set of probes was 3'-end labelled with deoxy[1',2',5,³H]cytidine 5' triphosphate (³H-dCTP,TRK.625, Amersham) and the other was 3'-end labelled with ³³P(α)dATP (BF1001), in a tailing reaction, using terminal deoxynucleotide transferase (Amersham).

As a result, the two target nucleic acids of the samples were clearly detected and discriminated from each other on the same samples.

### Example 4

In this example we show that our new method can be used to investigate a complex neurophysiological phenomenon such as long-term potentiation (LTP) and leads to innovative results that would be more difficult if not impossible to obtain without its use.
This brings additional data to show the efficiency of the method. This description of how our new method can be used in research activities in the specific neuroscience field of LTP is also provided here as an example of the utility of this method to bring new discoveries in any field of biological and physiological research.

### Physiological phenomenon studied.

Gene expression in neurons can vary in response to neuronal activation. In this study, to analyse the spatio-temporal dynamics of the transcriptional response of three genes following the induction of LTP within the entire rat dentate gyrus *in vivo*, we used our invention and compared it to two other long-standing and validated techniques: *in situ* hybridization with a single-probe and a single radioactive label analyzed on (i) an autoradiographic film and on (ii) an emulsion. This comparison of our invention with standard and well-validated techniques was aimed not only at validating it by itself (including its abilities to co-detect two different compounds in the same tissue section and to reliably quantify these compounds), but also at validating its research use in physiology and molecular biology. Zif268, Homer and syntaxin 1B genes were studied, and their regulated expression was examined at three times after the induction of LTP. Zif268 is an immediate early gene rapidly induced by LTP, Homer/Vesl is a molecule coupled to subunits of metabotropic glutamate receptors and syntaxin 1B is a protein of the exocytotic machinery involved in neurotransmitter release. These three genes were selected as they are known to be up-regulated at different times after the induction of LTP in the dentate gyrus.

Long-term potentiation is a form of enduring synaptic plasticity which has been widely studied as a candidate cellular mechanism for information storage in the brain. Its induction in the dentate gyrus of the hippocampus results in successive overlapping waves of transcription increase or decrease of a whole host of immediate early genes and effector genes in dentate granule cells, lasting from a few minutes to several days. This cascade of modifications of gene expression in particular cells leads to subsequent modifications of their function thus inducing modifications in cellular function.

A better understanding of the molecular behavior of such modified cells requires the identification of the genes involved and the characterizing of the amplitude (quantification) and time course of their expression, as well as their relative (inter-gene) expression levels. Although the temporal pattern of activation of several LTP-regulated genes has been characterized, very little is known about the spatial distribution of their regulated expression, and none about their relative levels of expression. To date, the analysis of gene expression in LTP has been largely limited to particular sub-regions of the dentate gyrus. Methods for establishing temporal and spatial profiles of numerous messenger RNA (mRNA) expression throughout the entire structure, coupled with fine cellular analysis, would make a large contribution to mapping cells, circuits and structures expressing particular mechanisms of plasticity such as LTP. Such methods require the ability to precisely quantify gene expression levels and compare inter-gene expression levels.

The invention reveals the spatial distribution and cell-specificity of both constitutive and regulated expression of three candidate genes, Zif268, Homer and syntaxin 1B, in the dentate gyrus. To analyze variations of the expression of the three mRNAs more precisely along the rostro-caudal axis of the dentate gyrus and across time, *in situ* hybridisation experiments were performed using our invention as well as the two other techniques listed above. Individual labelling of the mRNAs in serial sections throughout the entire dentate gyrus allowed the construction of a three-dimensional representation of their expression. In parallel, the invention based on double radioactive labelling was used to quantify two different mRNAs in the same brain section in other sets of *in situ* hybridization experiments in the same physiological paradigm. Both approaches revealed that LTP-regulated expression depends on the genes, on the position of the cells along the rostro-caudal axis of the dentate gyrus, and on time.

### Experimental procedure

### LTP induction

Male adult Sprague Dawley rats (Iffa Credo, France) weighing between 350 and 400 g were used. They were maintained in a temperature controlled colony room with free access to food and water. Rats in which LTP was induced were sacrificed either 30 minutes (Zif268), 3 hours or 5 hours (Homer and syntaxin 1B) post-tetanus. To examine the constitutive expression of the genes, control rats, subjected to pseudo-tetanus, were sacrificed at each of the three time points. The choice of time points for the analysis of expression of the three genes was determined by their kinetics of expression following LTP induction. As an immediate early gene, Zif268 mRNA is not up-regulated at 3 or 5 hours after LTP induction, whereas Homer and syntaxin 1B mRNAs are upregulated at these 2 time points, but their maximal expression does not occur at the same time.

Rats were anaesthetised with urethane carbamate (1.5 mg/kg), placed in a stereotaxic frame and maintained at a constant temperature with a thermostatically controlled heating blanket. Standard stereotaxic procedures, previously described, were used for unilateral induction of LTP of the perforant path - dentate gyrus synapses. In brief, recording electrodes (consisting of 2 nichrome wires (62 µm diameter) staggered 300 µm tip to tip, housed inside a stainless steel tube) were lowered into the hilus of the left dentate gyrus (Bregma -4.2 mm; Midline -2.5 mm). Multiunit activity and the field excitatory postsynaptic potential (EPSP) evoked by perforant path stimulation were monitored. A bipolar concentric stimulating electrode consisting of a stainless steel tube (150 µm diameter) placed inside a microtube (300 µm) was simultaneously lowered into the angular bundle of the left perforant path (Bregma -7.8 mm; Midline -4.4 mm). Final depths of both electrodes were adjusted to evoke a maximal positive-going field EPSP, which was allowed to stabilize for a further 30 minutes before starting the electrophysiological recordings.

Low frequency test pulses (100 µs, 0.033 Hz) were delivered to the perforant path throughout the entire experiment except when a tetanus or a pseudotetanus was delivered. Tetanic stimulation consisted of 6 trains of pulses (400 Hz, 20 ms) delivered every 10 seconds and repeated 6 times at 2-min intervals. Pseudotetanus followed the same pattern with single pulses instead of trains of pulses. The stimulation intensity was increased during tetanus or pseudotetanus to ensure maximal recruitment of fibres. Signals of the evoked responses were amplified and filtered (bandpass 0.1 Hz to 3 kHz) by a Grass preamplifier, displayed on a storage oscilloscope and fed into a computer for storage and off-line analysis via a CED interface. Stimulus intensities were selected for each rat to evoke a population spike height of approximately one third of its maximal height. Evoked responses were measured for one hour prior to the tetanus and for 30 minutes, 3 or 5 hours post-tetanus or post-pseudotetanus. Responses were stored as averages of 4, for later analysis of the maximal slope of the EPSP and the population spike height. All experimental procedures were carried out in accordance with the European Communities Council Directive (24.xi.1986) and with the guidlines of CNRS and the French Agricultural and Forestry Ministry (decree 87848, licence number: A91429). All efforts were made to minimize animal suffering and to use only the number of animals necessary to produce reliable scientific data.

### Tissue and DNA probe preparation

Approximately 280 coronal sections (20 µm-thick), covering the entire rostro-caudal extent of the hippocampus were cut using a cryostat. Sections were mounted on Superfrost plus slides and stored at -80°C. Antisense oligonucleotides were synthesized in-house on a Beckman Oligo 1000DNA synthesizer. Oligonucleotide sequences were complementary to rat mRNA-derived sequences available in published databases. The probes used were oligonucleotides complementary to sequences of Zif268 (45-mer oligonucleotide sequence: 5'-CCG TGG CTC AGC AGC ATC ATC TCC TCC AGT TTG GGG TAG TTG TCC-3'), syntaxin 1B (35-mer oligonucleotide sequence: 5'-GAT GTG TGG GGA GGG TCC TGG GGA AGA GAA GGG TA-3') and Homer (39-mer oligonucleotide sequence: 5'-GGT CAG TTC CAT CTT CTC CTG CGA CTT CTC CTT TGC CAG-3'). Probes were 3' end-labelled with α-³⁵S-deoxyadenosine triphosphate (Amersham, France) in a tailing reaction using terminal deoxynucleotide transferase (Amersham) according to the manufacturer's instructions. The specific disintegration activity after labelling was between 1x10⁸ and 3x10⁹ cpm/µg.

### In situ hybridisation with single radioactive labelling

Sections were post-fixed for 20 min in 4% paraformaldehyde in phosphate-buffered saline (PBS), washed 3 times for 10 min in PBS baths and dried in a 95° ethanol bath, immediately before hybridisation. The hybridisation solution was composed of 50% Amersham in situ hybridisation buffer, 40% formamide (Eurobio, France), 0.1M dithiothreitol (DTT) (Euromedex, France) and 0.5 mg/ml poly(A) (Roche, France). Probe stock solutions were diluted 1/100 in the hybridisation solution and 75 µl of the mixture was applied to each brain slice. Sections were incubated overnight at 50°C under Fuji parafilm coverslips, then washed twice for 15 min in 1X standard saline citrate (SSC)/10mM DTT at 53°C, twice for 15 min in 0.5X SSC/10mM DTT at 53°C and once in 0.5X SCC/10mM DTT at room temperature. They were then dried in a 95° ethanol bath and used to expose Amersham β-max film for one or two weeks. An autoradiographic scale was present on the film to determine the linear zone of labelling. Sections were then dipped in nuclear emulsion (Ilford K5 diluted in 2X SSC, France) for cellular analysis. Control experiments were performed either by displacing specific mRNA hybridisation with a 50-fold excess of unlabelled oligonucleotide or by using a sense oligonucleotide that yielded no signal in tissue sections.

### Three-dimensional reconstruction of in situ hybridisation experiments with single radioactive labelling

The autoradiograms were individually digitized by means of a CCD camera coupled to a digitization board, both driven by Samba software (Unilog, France). Regions of the dentate gyrus were analysed using a thick line drawn along the cell body layers of both lower and upper blades and the mean optical density per pixel (ODp) was measured. The pixel size was 23 x 23 µm². In *in situ* hybridisation experiments, one section in 5 (56 of 280 sections per rat) was hybridised with each probe. The hybridised sections were spaced every 100 µm for each rat and each probe. On the digitized images, the left and right dentate gyrus were segmented and a colour code was used to illustrate the differences of mRNA levels. *Volume*, a software described previously by Roesch et al. (J. Neurosci. Methods, 69 (1996), 197), was used for rigid registration of digitized images and subsequent assembly of segmentations generating three-dimensional wire-frame models of mRNA distribution.

### Double labelling in situ hybridisation experiments

The protocol used was the same as that described above except that 2 oligonucleotide probes were used: one labelled with α-³⁵S-deoxyadenosine triphosphate and the other with ³H-deoxycytosine triphosphate. Both probes were diluted 1/100 in the hybridisation solution¹⁵. Radioactive images were acquired with a Micro Imager (Biospace Mesures, Paris, France), a real time, high-resolution digital autoradiographic system. To analyse the double radiolabelling in the sections, a thin foil of scintillating paper was brought in contact with the sections. Beta particles emitted by the sections were identified by acquisition of the light spot emissions in the scintillating foil by a CCD, coupled to an image intensifer. The acquired results were displayed live on a computer. During acquisition, radioactive images can be saved to be analysed at any time. The end of the acquisition was chosen at a time such that the number of disintegrations followed through time was statistically satisfactory. The filter processing allowed discrimination and quantification in each pixel of the respective contributions of the two radioelements of significantly different energies. The outline of the cell body layer of the dentate gyrus of each brain section was delimited (as described above) and the number of disintegrations of β particles per area unit of this region was measured using β*-Vision* software (Biospace).

### Results

Constitutive expression of each gene in control conditions was homogeneous, but the spatial distribution of messenger RNA was heterogeneous along the rostro-caudal axis of the dentate gyrus following the induction of long-term potentiation, and different for each gene. In addition, the intensity of each gene-specific pattern of expression varied over time following the induction of long-term potentiation, as described below.

### Constitutive expression of Zif268, Homer and Syntaxin 1B.

To analyse the level of expression of each gene along the rostro-caudal axis of the dentate gyrus, *in situ* hybridisation experiments using individual labelling of the mRNAs on serial sections throughout the entire dentate gyrus were performed. A three-dimensional representation of mRNA expression was constructed with *Volume* software, as described above.

The spatial distribution of constitutive expression of Zif268, Homer and syntaxin 1B in both sides of the dentate gyrus in the control rats was first examined. The distribution of all three mRNAs along the entire rostro-caudal axis of the dentate gyrus on both sides was homogeneous and the three mRNAs were expressed at low constitutive levels (0-1 optical density unit per pixel (ODp) for Zif268; 0-3 ODp for Homer mRNA (Fig. 1A); 4-8 ODp for syntaxin 1B mRNA). There was no detectable change in the amount or distribution of these mRNAs after the pseudotetanus.

### Variations in the distribution of the three mRNAs following the induction of long-term potentiation

Following the induction of LTP, each of the three mRNAs showed a particular pattern in the stimulated side of the dentate gyrus: these three patterns were heterogeneous, whereas the distribution of mRNAs was homogeneous in the non stimulated side (Fig. 1B-1F).

Firstly, for Zif268 mRNA, the level of expression varied between the subregions in the stimulated side of the dentate gyrus, 30 minutes after the induction of LTP. The level was very low in the rostral part (maximum of 5 ODp; see Fig. 2A and 2B) and barely detectable in the caudal part (maximum of 1 ODp; see Fig. 2A). In the medial portion of the dentate gyrus, Zif268 mRNA was more abundant; in the anterior part of the medial region, there was more mRNA in the lower blade (between 15 and 45 ODp) than the upper blade (between 0 and 20 ODp) (see Fig. 2C), whereas in the posterior part of the medial dentate gyrus, the upper blade (between 15 and 30 ODp) gave a stronger mRNA signal than the lower blade (between 0 and 10 ODp; see Fig. 2A and 2D).

For Homer and syntaxin 1B mRNAs, the distribution of both mRNA species along the rostro-caudal axis differed three hours after the induction of LTP. Syntaxin 1B mRNA showed a greater level of expression at the rostral and caudal parts of the dentate gyrus (both subregions between 15 and 25 ODp) and in only a few medial sections of the dentate gyrus, than in the other regions (Fig. 1C). The mRNA for Homer, however, was most abundant in the medial part of the dentate gyrus (between 15 and 25 ODp) and to a much lesser extent at the rostral and caudal ends (between 5 and 10 ODp; Fig. 1D).

Five hours after the induction of LTP, the level of syntaxin 1B mRNA was, in general, higher along the entire rostro-caudal axis of the dentate gyrus than it was 3 hours after induction of LTP (Fig. 1C and 1E). In contrast, the expression of Homer was lower at 5 hours than 3 hours, in agreement with preliminary experiments suggesting that the level of LTP-induced expression of Homer tends to peak between 1 and 3 hours after induction of LTP (Fig. 1F). At all locations along the axis, the levels of both syntaxin 1B and Homer mRNAs were always lower in the non-stimulated side than the stimulated side of the dentate gyrus. Contrary to Zif268 mRNA, no differential distribution between the two blades of the dentate gyrus was found for Homer or syntaxin 1B mRNAs.

Note that the data in figures 1E, IF, 3, 4 and 5 but not 1A-1D are from the same animal, which underwent LTP induction and was sacrified five hours after LTP induction. The data in figure 2 are from the animal used for figure 1B.

### Spatio-temporal heterogeneity of long-term potentiation-induced gene expression confirmed by emulsion dripping and double labelling

All the brain sections processed for *in situ* hybridisation were dipped in radiographic emulsion. The spatial distribution of expression of the genes was then assessed by silver grain counting. Gene expression assessed by grain counting was in agreement with that assessed by densitometry on autoradiographic films (Fig. 3F-3I and 4F-4I).

The spatial heterogeneity of mRNA expression following LTP induction was confirmed by the invention (novel method of double radioactive labeling) described in 'Experimental procedure, Double labeling in situ hybridization experiment': a ³⁵S-labelled probe was used to detect Homer mRNA and a ³H-labelled probe was used to detect syntaxin 1B mRNA in the same sections. This novel technique was applied to 16 sections from various locations along the rostro-caudal axis of the dentate gyrus from each brain. In the rats in which LTP was induced, we chose the locations at which variations of Homer and syntaxin 1B mRNA expression were clearly different in the 3-D reconstruction. The mRNA levels determined by double labelling sections were in agreement with the results, in the same brains, of single-labelling *in situ* hybridisation using two markers on adjacent brain sections (Fig. 3 and 4).

The cellular heterogeneity observed with the three methods (autoradiographic film, emulsion and double radioactive labelling) is illustrated in figures 3 and 4. These figures show sections located at two positions (we called α and β) of the stimulated dentate gyrus from a rat in which LTP was monitored for 5 hours. Note that in double radioactive labelling experiments, figures 3B and 4B result from the same brain section at the α position of the dentate gyrus: figure 3B corresponds to ³H Beta disintegration of the syntaxin 1B probe, and figure 4B to ³⁵S Beta disintegration of the Homer probe. Figures 3C and 4C result also from the same brain section but at the β position of the dentate gyrus. Five hours after LTP induction, the Homer mRNA signal (double labelling method) was about twice as high at the α position of the stimulated side of the dentate gyrus than at the β position. The amount of syntaxin 1B mRNA at the α position was 0.78 times that at the β position (Fig. 5 and Table 1).

**Table 1. Ratios of mRNA abundance at position α to that at postion β of the dentate gyrus for Homer and syntaxin 1B obtained by the single and double labelling methods: Values are ratios of mRNA abundance for the α to β sections of the dentate gyrus (DG) from a brain in which LTP was monitored for 5 hours (shown in figures 3 and 4). The ratios obtained after LTP were similar with the two methods of hybridisation, and the levels for both syntaxin 1B and Homer mRNA were equal in the non-stimulated dentate gyrus at both the α and β levels, with ratios close to 1, evidence that there was no modulation in the control side.**

| mRNA species | Homer | | Syntaxin 1B | |
|---|---|---|---|---|
| side of DG | stimulated | non stimulated | stimulated | non stimulated |
| single labelling | 2.10 | 1.00 | 0.80 | 0.98 |
| double labelling | 2.08 | 0.96 | 0.78 | 0.96 |

The ratios obtained from the double labelling, including those 5 hours after LTP induction, were consistent with those observed in the previous *in situ* hybridisation experiment investigating the different markers on adjacent sections. This confirms the spatial heterogeneity of mRNA expression (Table 1). In addition, these results were in accordance with those from sections dipped in emulsion (Fig. 3F-3I and 4F-4I).

### Spatial heterogeneity of mRNA expression within the same coronal section of the dentate gyrus following the induction of long-term potentiation

Double labelling was used to distinguish populations of cells with similar or dissimilar transcriptional responses within a single coronal brain section (Fig. 5). For example, at position α of the dentate gyrus, Homer and syntaxin 1B mRNA levels were studied along the granule cell layer. Some cell populations expressed syntaxin 1B mRNA weakly and Homer mRNA strongly, whereas others showed the opposite pattern (Fig. 5A, 5C, 5E and 5G). This demonstrates spatial heterogeneity of LTP-induced gene expression within a coronal brain section in addition to the spatial heterogeneity along the rostro-caudal axis of the dentate gyrus.

### Discussion

These results confirm the efficiency and reliability of the invention, as it provided results that were in accordance with those from experiments using single-labelling *in situ* hybridization analyzed on autoradiographic films and emulsion: after the induction of LTP, the different genes studied were differentially modulated in the dentate gyrus, depending on their position along the rostro-caudal axis, on the gene and on time.

Moreover, by making it possible to compare the expression of two genes on a same coronal section, the invention specifically provided additional results, namely evidence that, at a same location along the rostro-caudal axis, different cell populations present different patterns of expression of Homer and Syntaxin 1B after LTP induction. This result would have been impossible to obtain by any other method. Indeed, in the other *in situ* hybridization methods, the different genes have to be studied on different brain sections, making it impossible to compare their expression in exactly the same cell populations.

Globally, the use of this invention shows that three selected mRNAs, each with homogeneous constitutive expression in the dentate gyrus, have very different spatial and temporal patterns of expression following LTP induction. It also demonstrates the diversity of cellular responses to the induction of LTP within a single brain structure. These results suggest that there are several molecular mechanisms of long-term potentiation, differing from one cluster of cells of the dentate gyrus to another, or that the different signaling pathways involved in long-term potentiation are used with varying efficiencies by different cells. In physiological terms, this variation in the efficiency of signaling pathways or in the molecular mechanisms involved is likely to be related to differences in number and/or position of the synapses undergoing a change in strength on a given granule cell, and on the overall amount of synaptic drive. In all, this reveals the existence of overlapping temporal waves of gene expression with a cellular specificity in the dentate gyrus and highlights the temporal and spatial complexity of the mechanisms involved in LTP. This also suggests that the differential integration of excitatory and inhibitory inputs on neurons that is reflected in the overall response of a cell has, in the case of a change in synaptic strentgh as a result of LTP, important consequences downstream in intracellular signaling to the nucleus, resulting in a differential transcriptional response.

These concepts are clearly new with regard with LTP induction in the dentate gyrus. These results illustrate the potential of the methods developed in this invention for analysing the dynamics of regulated gene expression spatially and temporally in the brain, and generate new hypothesis in neuroscience's complex phenomena.

The heterogeneity observed cannot simply result from the position of the stimulating electrode, differentially affecting subregions of the dentate gyrus. To stimulate the maximum number of fibres of the perforant path projecting onto granule cells, care was taken to position the stimulating electrode in the angular bundle, a region in which afferents arising from most of the input layers of the entorhinal cortex come together. As stated previously, the closer to the angular bundle one stimulates, the more widespread the activation of the dentate gyrus along the longitudinal axis. Moreover, although it could be predicted to result in a certain degree of spatial heterogeneity, it cannot by itself explain the gene-specificity of the cell response observed here on the same brain sections, as shown by the invention.

### Technological considerations

Until now, 3-D reconstruction has been used only to model structures or to localize molecules or particular cells within a organ structure (Nat. Genet. 25 (2000), 147). The invention improves 3-D reconstruction by adding the new possibility of quantitative analysis of gene expression within an entire organ structure (here a brain structure). This has never been carried out previously.

To date, *in situ* hybridisation using non-radioactive probes has allowed detection of several mRNAs in the same tissue section (alkaline phosphatase/peroxidase) or in the same cell (fluorescence). However these methods are only qualitative. In contrast, radioactive labelling can be used to measure the level of gene expression, but until now only one gene could be analysed at a time. The invention makes it possible to simultaneously use two probes labelled with radioelements of significantly different energies, such as ³H and ³⁵S, and to filter a double radioactive image acquired by a Micro Imager into two subimages, each one representing the specific hybridisation of one probe. The Micro Imager is a real time, high resolution digital autoradiography system. Its direct particle counting principle in real time avoids the problems of underexposure and saturation, and the novel method of processing allows local discrimination and quantification of the contributions of each of label for each pixel in the same brain section. Moreover the high dynamic range (10⁴) of the Micro Imager allows the comparative analysis of strong and weak signals on the same tissue section, and as such it is appropriate for studying the expression profiles frequently observed in the central nervous system. Very small variations of expression for two different mRNA species can therefore be measured and distinguished within a single section with a resolution of 15-20 µm. With a pixel size of 23x23 µm² and a resolution of 15-20 µm, the differences in the levels of expression of two mRNAs between pixels within the same brain structure can be attributed to the existence of cells or clusters of cells that have different responses in mRNA expression, assuming that the mRNAs studied are expressed only in neuron cell bodies, as it is the case for the three mRNAs studied here.

The identification and characterizing of the populations of activated cells in the whole dentate gyrus according to their level of gene expression will help to elucidate the behaviour of the cells affected by the induction of LTP. Experiments with a larger number of genes allow easier analysis of the mechanisms underlying LTP. By coupling the double radioactive labelling technique with three-dimensional reconstruction, twice as many markers can be tested. In this example, each probe was hybridised every 5 sections and this was sufficient to characterise the heterogeneity of cellular response to LTP. Therefore, the invention makes it possible to quantitatively study 10 markers per rat throughout the hippocampus and establish their individual spatial profiles of expression (instead of 5 markers previously). In summary, the availability of a technique for investigating the simultaneous expression patterns of several genes per brain, makes it possible to map the distribution of markers of synaptic plasticity and construct images of activated cells, circuits and brain structures in individual animals.

### Exemple 5

A better understanding of biological phenomena involving modulations of gene expression requires the quantitative analysis of the expression of several genes within a same structure or sub-structure of the organ (tissue) of interest. The invention allows the quantification of two different messenger RNA (mRNA) species in the same tissue section simultaneously. Two probes labelled with radioelements of significantly different energies (³H and ³³P or ³⁵S), were simultaneously used to detect two different mRNA species. Radioactive images corresponding to the detected mRNA species were acquired with a Micro Imager, a real time, high-resolution digital autoradiography system. An algorithm was used to process the data such that the initial radioactive image acquired was filtered into two sub-images, each representative of the hybridisation result specific to one probe. This novel method allows the local discrimination and the quantification of the respective contributions of each label to each pixel and can thus be used for quantitative analysis of two mRNAs with a resolution of 15-20 µm.

*In situ* hybridisation (ISH) is now a routine method for the detection of genetic material in cells or tissues. It is used in a large number of biological fields such as anatomy, cellular biology and regulation of gene expression^{2, 13, 14}. Since 1990, the characterisation of numerous genes and complementary DNAs, and the rapid development of molecular biology techniques have led to ISH becoming widely used, powerful and user friendly. For example, this technique has become of great importance for localising individual cells that contain a particular specie of mRNA within the complex and heterogeneous substance of the nervous system. The anatomical data obtained by ISH are very accurate and provide regional, cellular and sub-cellular patterns of gene expression^{4, 6, 9, 11}. However, these analyses suffer from several drawbacks particularly for quantitative analysis of more than one gene. Fluorescent labelling is generally used for the simultaneous visualisation of the expression of several genes within a single cell¹². However, fluorescence does not allow quantification and is not sensitive enough to detect small changes in gene expression or to detect rare mRNAs (e.g. low-abundant mRNAs). Quantitative data about the level of gene expression can only be obtained using radioactively labelled probes, but such analyses are only possible for one mRNA specie at a time^{3, 6}. Therefore, a technique able to detect and quantify several mRNAs species in the same tissue section within a single cell is of great value.

In 1994, a new *in situ* hybridisation approach was described. It was based on the direct detection of radioactive emission, by using the high resolution of a radio imager to analyse mRNA expression in brain tissue sections. The main advantage of this approach over standard autoradiographic approaches is the possibility of quantifying mRNA in real time and with a high dynamic range (10⁴), leading to cellular resolution in shorter delays. Recently the use of this device by developing adequate signal acquisition and processing algorithms to discriminate different radioactive-emission spectra obtained simultaneously have been improved. The present invention demonstrates simultaneous *in situ* hybridisation of two radioactive probes on the same tissue section, each probe being labelled with radioelements of significantly different energies (³H and ³³P or ³⁵S). It also demonstrates that this allows quantitative analysis of two mRNAs in a single section.

### Materials and Methods

### Tissue preparation

One male adult Sprague Dawley (Iffa Credo, L'Arbresle, France) weighing between 350 and 400 g was anaesthetised with urethane carbamate (1.5 mg/kg), and placed in a stereotaxic frame for electric stimulations. The animal was sacrificed and its brain was extracted and frozen in isopentane at -60°C. Coronal sections (20 µm-thick) were cut using a cryostat at -22°C. Sections were mounted on Superfrost plus slides and stored at - 80 °C.
All experimental procedures were carried out in accordance with the European Communities Council Directive (24.xi.1986) and with the guidlines of the CNRS and the French Agricultural and Forestry Ministry (decree 87848, licence number: A91429).

### Double radioactive in situ hybridisation

Two oligonucleotide probes were used for these experiments: one is complementary to part of the syntaxin 1B sequence (35-mer oligonucleotide sequence: 5'-GAT GTG TGG GGA GGG TCC TGG GGA AGA GAA GGG TA-3') and the other to part of the Homer sequence (39-mer oligonucleotide sequence: 5'-GGT CAG TTC CAT CTT CTC CTG CGA CTT CTC CTT TGC CAG-3'). Oligonucleotides were synthesized in-house on a Beckman Oligo 1000DNA synthesizer. The probes were 3' end-labelled with ³⁵S-deoxyadenosine triphosphate (Amersham, Orsay, France) or ³H-deoxycytosine triphosphate (Amersham) in a tailing reaction, using terminal deoxynucleotide transferase (Amersham) according to the manufacturer's instructions. The specific disintegration activity after labelling was between 1x10⁸ and 3x10⁹ cpm/µg for each probe.
Coronal brain sections (20 µm-thick) were post-fixed in 4% paraformaldehyde in phosphate-buffered saline (PBS), then washed 3 times for 10 min in PBS baths and dried in a 95°C ethanol bath, immediately before hybridisation. The hybridisation solution was composed of 50% Amersham in situ hybridisation buffer, 40% formamide (Eurobio, Les Ulis, France), 0.1M dithiothreitol (DTT) (Euromedex, Souffel Weyersheim, France) and 0.5mg/ml poly(A) (Roche, Saint Quentin Fallavier, France). Both probes were diluted 1/100 in the hybridisation solution and 75 µl of the mixture was applied to each brain slice. Sections were incubated overnight at 50°C under Fuji parafilm coverslips, then washed twice for 15 min in 1X standard saline citrate (SSC)/10mM DTT at 53°C, twice for 15 min in 0.5X SSC/10mM DTT at 53°C and once in 0.5X SCC/10mM DTT at room temperature and then dried in a 95° ethanol bath. Radioactive signals from the sections were acquired with a Micro-Imager (Biospace Mesures, Paris, France), which is a real time, high-resolution digital autoradiography system.

### Imaging equipment for radiolabelled tissue sections

To analyse the double radiolabelling in the sections, a thin foil of scintillating paper is brought into contact with the sections. Beta particles emitted by the sections are identified through acquisition of the light spot emissions in the scintillating foil by a CCD camera that is coupled to an image intensifier. The result of the acquisition is displayed live on a computer. During the acquisition, radioactive images can be saved at any time to be analysed. Acquisition is stopped once the number of acquired disintegrations is statistically sufficient. The filter processing allows discrimination and quantification in each pixel of the respective contributions of the two radioelements of significantly different energies.

### Results

To show the feasibility of simultaneous in situ hybridisation of two radioactive probes on a same section, electric stimulations were used for neuronal activation in one side of a rat brain. The expression of the two genes studied, Homer and syntaxin 1B, that are differentially regulated, was followed.

The principle of the double labelling ISH technique is illustrated in figure 7 . ³⁵S-dATP and ³H-dCTP were chosen to label two different probes which were simultaneously hybridised to a single tissue section. Micro Imager was used to acquire the signal from the hybridised section in a single step. The initial image was consequently filtered to segregate the image corresponding to ³H Beta disintegrations (Figure 8C) from that corresponding to ³⁵S Beta disintegrations (Figure 8E). The quantitative data for both ³H and ³⁵S labelling were incorporated into a single image (Figure 8A). In figure 8A, green corresponds to the cells that contain only the mRNA detected by the ³H-labelled probe, red to those that contain only the mRNA detected by the ³⁵S-labelled probe, and yellow to those that contain both.

To control the filtering segregating ³⁵S-beta disintegrations from the ³H-ones, three control dots were spotted by hand on the slide. The dots contained the ³H-labelled probe (200 cpm), a mix of the ³H- (200 cpm) and the ³⁵S- (200 cpm) labelled probes. All three spots are observed in the image with both labels (Figure 8A) and only two dots after filtering, as expected (Figure 8C, 8E). The quantification of the radioactivity emitted by each dot before and after filtering gave values in accordance with the amount of radioactivity spotted.

The expression of the two mRNAs along a line drawn on the section is quantitatively analysed on figure 8 for illustration. The respective contribution of each label to each pixel along this line is shown on graphs (Figures 8B, 8D, 8F). From the graphs, cells that differentially expressed the two mRNA species are clearly identified and others expressed them at a similar level. This novel method allows quantitative comparison of the expression of these mRNAs in different cells. For example, the cells indicated by arrow 4 expressed about 5 times as much mRNA hybridising with the ³H-labelled probe as the cells indicated by arrow 3. They also contain large amounts of mRNA detected by the ³⁵S-labelled probe whereas the amount in the cells indicated by arrow 3 is barely detectable (see Figure 8).

### Discussion

Numerous ISH protocols have been developed. They use either enzymatically synthesised RNA and DNA probes or chemically synthesised DNA probes ("oligodeoxynucleotide" probes). Standard protocols use either non-radioactive or radioactively labelled probes. The method of signal detection to be used depends upon the required level of resolution and sensitivity but also upon the physiological context^{2, 13, 14}_{.}

Non-radioactive probes are mainly used for anatomical analyses of gene expression, because they provide the greatest spatial resolution and they allow detection of several mRNAs in the same tissue section (peroxidase/ alcaline phosphatase), in the same cell (fluorescence)¹⁰, and even in confocal microscopy field for sub-cellular discrimination¹². Moreover, the results are obtained rapidly (1 or 2 days). However non-radioactive probes do not provide quantitative results concerning the level of gene expression, and are useful only for the identification of the cells that contain a particular mRNA or DNA¹⁴.

In contrast, radioactive labelling allows precise measurement of the level of gene expression^{2, 13}. Various isotopes can be used for labelling probes such as for example ³H, ³⁵S, ³³P and ³²P. Various methods are used to quantify mRNA: classical autoradiographic methods (film and emulsion)^{3, 4}; indirect detection through storage in phosphor-screens⁵ and direct detection through a solid scintillator sheet coupled to a CCD camera (µImager)^{7, 8}.
For analysis of the regional distribution of mRNA, storage phosphor-screens (resolution of 80 µm (³H) and 180 µm (³⁵S/¹⁴C)) and autoradiographic films (20-30µm) allow quantification of signals with exposure times of several days to weeks for films, and 8 fold less for storage phosphor-screens. To detect mRNA in individual cells, the hybridised sections are usually dipped into nuclear emulsion: the amount of the mRNA can be quantified at a cellular level by counting grains. The exposure time required for this technique is often long, from several weeks to several months depending on the amount of the mRNA in the tissue¹³. These three radioactive techniques can not be used for simultaneous analysis of two mRNA species in a single section.
Here, we demonstrate that the invention makes it possible to analyse the samples simultaneously with two probes with double radioactive labelling and that the Micro Imager, in contrast to other techniques, allows quantitative co-detection. Moreover this is performed in real time, with a high dynamic range (10⁴), satisfactory resolution (15 µm) and exposure times 10 times shorter than autoradiographic films and 50 times shorter than emulsion. The high dynamic range of the Micro Imager allows the comparative analysis of weak and strong signals on the same tissue section, such expression profiles being commonly observed in the central nervous system. The accuracy is better than 5% without underexposure owing to the direct particle counting principle of the instrument in real time such that acquisition can be halted at the appropriate time. Very small variations of expression for several genes can therefore be measured with high accuracy on a same section.

Our in situ hybridisation experiments, performed with two different labelled probes (here ³H and ³⁵S), demonstrate the feasibility of double labelling procedures to study simultaneously the expression of different mRNA species in a single tissue section. To our knowledge, this is the first report of ISH specific detection and quantification of more than one transcript, allowing the comparison of the expression of several genes at the cellular level. The findings with this approach were compared with those obtained by independent single labelling ISH experiments on adjacent sections. As expected, the expression patterns observed were qualitatively and quantitatively similar which validates the invention.

Two probes hybridised on the same section can only be distinguished from each other if the radioisotopes used to label them have different emission-energy spectra. We labelled one probe with ³H and the other with either ³³P or ³⁵S. ³⁵S and ³³P have similar spectra, but different half-lives. However, the ³H energy spectrum is clearly different from those of ³³P and ³⁵S ³⁶. The disintegration half-life of ³H is more than 1 log (10 times) longer than those of ³³P and ³⁵S. Therefore the frequency of disintegration events is much lower with ³H for a given amount of isotope and is the reason for the long exposure times commonly used with ³H labelling (such as in autoradiography techniques). For the double labelling technique, it is crucial that the both labelling signals are simultaneously acquired. However, when separately adapting the probe-labelling procedures for each of these radioisotopes, we were able to establish a protocol in which acquisition times were equivalent for both ³H and the other isotopes. This allows a single acquisition of the images corresponding to the ³H and ³³P (or ³⁵S) isotopes. Discriminating a third isotope, such as ³²P, from both ³H and ³³P/³⁵S is also feasible with adequate adaptation of signal acquisition software.
*In situ* hybridisation has already made a huge contribution to our understanding of how cellular events interrelate and how mRNA is organised, spliced and transported. Double radioactive detection may now further improve the power of this approach and is suitable for gene expression screenings on tissue sections. It may also allow novel types of experiments, for example co-detection of a mRNA specie (with a radiolabelled nucleotide probe) and a protein (with a ¹²⁵I-radiolabelled antibody). Furthermore, the co-detection of two radioactively labelled compounds of a biological tissue could be used in conjunction with the detection of other molecules using non radioactive labelled probes or reagents. This would allow the quantitative and qualitative analysis of 5 markers on a single tissue section, two of them (or more in the future) being labelled with radioactive molecules.

### REFERENCES

1. Charon Y, Bendali M, Cuzon JC, Leblanc M, Mastrippolito R, Tricoire H, Valentin L (1990) HRRI: a high-resolution β- imager for biological applications. Nucl Instr and Meth A262: 179-186
2. Chesselet MF (1990) In situ hybridization histochemistry. CRC Press Inc, Boca Raton, Florida
3. Dumas S, Pequignot JM, Ghilini G, Mallet J, Denavit-Saubie M (1996) Plasticity of tyrosine hydroxylase gene expression in the rat nucleus tractus solitarius after ventilatory acclimatization to hypoxia. Brain Res Mol Brain Res 40: 188-94.
4. Dumas S, Javoy-Agid F, Hirsch E, Agid Y, Mallet J (1990) Tyrosine hydroxylase gene expression in human ventral mesencephalon: detection of tyrosine hydroxylase messenger RNA in neurites. J Neurosci Res 25: 569-75.
5. Ito T, Suzuki T, Lim DK, Wellman SE, Ho IK (1995) A novel quantitative receptor autoradiography and in situ hybridization histochemistry technique using storage phosphor screen imaging. J Neurosci Methods 59: 265-71
6. Javoy-Agid F, Hirsch EC, Dumas S, Duyckaerts C, Mallet J, Agid Y (1990) Decreased tyrosine hydroxylase messenger RNA in the surviving dopamine neurons of the substantia nigra in Parkinson's disease: an in situ hybridization study. Neuroscience 38: 245-53
7. Laniece P, Charon Y, Cardona A, Pinot L, Maitrejean S, Mastrippolito R, Sandkam B, Valentin L (1998) A new high resolution radioimager for the quantitative analysis of radiolabelled molecules in tissue section. J Neurosci Methods 86: 1-5
8. Laniece P, Charon Y, Dumas S, Mastrippolito R, Pinot L, Tricoire H, Valentin L (1994) HRRI: a high resolution radioimager for fast, direct quantification in in situ hybridization experiments. Biotechniques 17: 338-45
9. Le Guellec P, Dumas S, Volle GE, Pidoux E, Moukhtar MS, Treilhou-Lahille F (1993) An efficient method to detect calcitonin mRNA in normal and neoplastic rat C-cells (medullary thyroid carcinoma) by in situ hybridization using a digoxigenin-labeled synthetic oligodeoxyribonucleotide probe. J Histochem Cytochem 41: 389-95
10. Nederlof PM, van der Flier S, Wiegant J, Raap AK, Tanke HJ, Ploem JS, Van der Ploeg M (1990) Multiple fluorescence in situ hybridization. Cytometry 11: 126-31
11. Okamura H, Abitbol M, Julien JF, Dumas S, Berod A, Geffard M, Kitahama K, Bobillier P, Mallet J, Wiklund L (1990) Neurons containing messenger RNA encoding glutamate decarboxylase in rat hypothalamus demonstrated by in situ hybridization, with special emphasis on cell groups in medial preoptic area, anterior hypothalamic area and dorsomedial hypothalamic nucleus. Neuroscience 39: 675-99
12. aratore C, Suter U, Sommer L (1999) Embryonic gene expression resolved at the cellular level by fluorescence in situ hybridization. Histochem Cell Biol 111: 435-43
13. Valentino KL, Eberwine JH, Barchas JD (1987) In situ hybridization. Applications to Neurobiology. Oxford University Press. New York Oxford, 1-242
14. Wilkinson DG (1994) In situ hybridization: A practical approach. IRL Press, Oxford, UK

## Claims

1. A method of simultaneously detecting target nucleic acids in a biological sample, comprising:
a) contacting the biological sample with at least two sets of radioactive probes, the probes of the first set being specific for a first target nucleic acid and labelled with a first radio-label, and the probes of the second set being specific for a second target nucleic acid and labelled with a second radio-label, the at least two sets of probes having a specific disintegration activity not differing by more than about three times from each other(s), preferably not by more than about two times, and
b) simultaneously detecting said first and second target nucleic acids in the biological sample by assessing in situ the formation of hybrids between the probes and the sample.

2. The method of any one of claim 1, wherein both sets of probes have a specific disintegration activity comprised between about 5.10⁷ and 5.10¹⁰ cpm/µg.

3. The method of claim 2, wherein both sets of probes have a specific disintegration activity comprised between about 10⁸ and 10¹⁰ cpm/µg, more preferably between about 5.10⁸ and 5.10⁹ cpm/µg.

4. The method of any one of the preceding claims, wherein the probes are DNA molecules between 15 and 2000 base-pair long, more preferably between 15 and 500 base-pairs-long.

5. The method of claim 4, wherein the probes are single stranded DNA oligonucleotides between 15 and 500 bases long.

6. The method of one of claims 1 to 3, wherein the probes are RNA molecules, between 15 and 3000 bases long.

7. The method of any one of the preceding claims, wherein the probes are labelled by a 3' radioactive tracer, preferably a 3', 5-100 long, radiolabelled nucleic acid tail.

8. The method of any one of claims 1 to 7, wherein the probes are labelled by a 5' radioactive tracer.

9. The method of any one of claims 1 to 8, wherein the probes comprise, in their sequence, radioactive nucleotides.

10. The method of any one of the preceding claims, wherein the two sets of probes are comprised of nucleic acids of the same nature, for instance oligonucleotide probes.

11. The method of any one of the preceding claims, wherein the probes of the two sets comprise a 3' radioactive tracer or a 5' radioactive tracer or comprise, in their sequence, radioactive nucleotides, the probes of the two sets comprising more preferably a 3' radioactive tracer.

12. The method of any one of the preceding claims, wherein the first and second radiolabel have a different emission-energy spectra.

13. The method of claim 12, wherein the first set of probes is labelled with tritium and the second set of probes is labelled with a radioisotope selected from ³⁵5, ³³P, ³²P and ¹²⁵I.

14. The method of any one of the preceding claims, wherein the two sets of probes are contacted simultaneously or sequentially with the biological sample and/or the target nucleic acids are detected by assessing simultaneously in situ the formation of hybrids between the two sets of probes and the sample.

15. The method of any one of the preceding claims, wherein essentially the same amount of the two sets of probes is used.

16. The method of any one of the preceding claims, wherein the biological sample is a mammalian tissue sample, preferably a tissue section.

17. The method of any one of the preceding claims, wherein several biological samples are contacted in parallel.

18. The method of any one of the preceding claims, wherein the samples are deposited on one or several supports, preferably glass support.

19. A method of simultaneously detecting target nucleic acids in several biological samples, comprising:
a) providing biological samples on one or several supports, preferably glass supports,
b) contacting, in parallel, each of the biological samples on the support(s) with at least two sets of radioactive probes, the probes of the first set being specific for a first target nucleic acid and labelled with a first radio-element, and the probes of the second set being specific for a second target nucleic acid and labelled with a second radio-element, the at least two sets of probes having specific disintegration activity not differing by more than about three times from each other(s), preferably not by more than about two times, and
c) simultaneously detecting said first and second target nucleic acids in each of the biological samples by assessing in situ the formation of hybrids between the probes and the samples.

20. A method of simultaneously detecting target nucleic acids in a biological sample,
wherein the biological sample is contacted, in parallel with the following at least two sets of probes:
a) probes of a first set specific for a first target nucleic acid and labelled with a first radio-label and probes of a second set specific for a second target nucleic acid and labelled with a second radio-label,
b) probes of the first set specific for the first target nucleic acid and labelled with the second radio-label and probes of the second set specific for the second target nucleic acid and labelled with the first radio-label,
wherein the at least two sets of probes have a specific disintegration activity not differing by more than about three times from each other(s), preferably not by more than about two times and wherein the method further comprises simultaneously assessing in situ the formation of hybrids between the probes and the samples.

21. A method for simultaneously comparing target gene expression in at least two biological samples, comprising:
a) contacting, in parallel, the biological samples with at least two sets of radioactive probes, the probes of the first set being specific for a first target nucleic acid and labelled with a first radio-element, and the probes of the second set being specific for a second target nucleic acid and labelled with a second radio-element, the at least two sets of probes having specific disintegration activity not differing by more than about three times from each other(s), preferably not by more than about two times,
b) simultaneously assessing in situ the formation of hybrids between the probes and the samples, and
c) quantitatively comparing target gene expression in said samples by simultaneously comparing the relative amount of hybrids formed between the samples.

22. The method of any one of the preceding claims, wherein one of the sets of probes is specific for a control reference nucleic acid.

23. The method of any one of the preceding claims, wherein assessing hybrid formation comprises (i) washing the unbound probe and (ii) detecting radioactivity on the sample.

24. The use of two radioactive probes with different nucleic acid sequences and different radioactive labels, the two probes having specific disintegration activity not differing by more than about three times from each other(s), preferably not by more than about two times, for simultaneous in vitro or ex vivo gene expression analysis on a same biological sample according to any of claims 1-26 of the underlying application.

25. A method of any one of claims to 23, further comprising contacting the biological sample(s) with a non-radioactive probe and/or an affinity reagent to detect additional target nucleic acid(s), polypeptide(s) or cellular component(s).

26. A method for simultaneous detection or quantification of at least two target polypeptides of a cell or tissue, in several biological samples, comprising:
a) contacting, in parallel, the biological samples with at least two sets of differently radio-labelled antibodies, the antibodies of a first set being specific for a first target polypeptides and labelled with a first radio-element, and the antibodies of a second set being specific for a second target polypeptide and labelled with a second radio-element, the at least two sets of antibodies having specific disintegration activity not differing by more than about three times from each other(s), preferably not by more than about two times,
b) assessing in situ the formation of hybrids between the antibodies and the samples, and optionally
c) quantitatively comparing target polypeptide expression in said samples by comparing the relative amount of hybrids formed between the samples.

## Patentansprüche

1. Ein Verfahren für das gleichzeitige Nachweisen von Zielnukleinsäuren in einer biologischen Probe, umfassend:
a) Das In-Kontakt-Bringen der biologischen Probe mit mindestens zwei Gruppen radioaktiver Sonden, wobei die Sonden der ersten Gruppe spezifisch für eine erste Zielnukleinsäure sind und mit einer ersten radioaktiven Markierung markiert sind, und
wobei die Sonden der zweiten Gruppe spezifisch für eine zweite Zielnukleinsäure sind und mit einer zweiten radioaktiven Markierung markiert sind, wobei die mindestens zwei Sondengruppen eine spezifische Zerfallsaktivität haben, die sich nicht mehr als ungefähr um das dreifache, vorzugsweise nicht mehr als ungefähr um das zweifache voneinander unterscheiden, und
b) das gleichzeitige Nachweisen der ersten und zweiten Zielnukleinsäuren in der biologischen Probe durch Auswerten der *in situ* Hybridbildung zwischen den Sonden und der Probe.

2. Das Verfahren nach Anspruch 1, wobei beide Sondengruppen eine spezifische Zerfallsaktivität haben, die zwischen ungefähr 5 x 10⁷ und 5 x 10¹⁰ cpm (Zerfälle pro Minute)/µg umfasst.

3. Das Verfahren nach Anspruch 2, wobei beide Sondengruppen eine spezifische Zerfallsaktivität haben, die zwischen ungefähr 5 x 10⁸ und 5 x 10¹⁰ cpm (Zerfälle pro Minute)/µg, mehr bevorzugt zwischen ungefähr 5 x 10⁸ und 5 x 10⁹ cpm (Zerfälle pro Minute)/µg umfasst.

4. Das Verfahren nach einem der vorhergehenden Ansprüche, wobei die Sonden DNA-Moleküle sind, die zwischen 15 und 2000 Basenpaare lang, mehr bevorzugt zwischen 15 und 500 Basenpaare lang sind.

5. Das Verfahren nach Anspruch 4, wobei die Sonden einzelsträngige DNA-Oligonukleotide sind, die zwischen 15 und 500 Basen lang sind.

6. Das Verfahren nach einem der Ansprüche 1 bis 3, wobei die Sonden RNA-Moleküle sind, die zwischen 15 und 3000 Basen lang sind.

7. Das Verfahren nach einem der vorhergehenden Ansprüche, wobei die Sonden mit einem 3'-radioaktiven Indikator markiert sind, der vorzugsweise ein 3'-, 5 - 100 Basen langer, radioaktiv markierter Nukleinsäureschwanz ist.

8. Das Verfahren nach einem der Ansprüche 1 bis 7, wobei die Sonden mit einem 5'-radioaktiven Indikator markiert sind.

9. Das Verfahren nach einem der Ansprüche 1 bis 8, wobei die Sonden in ihrer Sequenz radioaktive Nukleotide umfassen.

10. Das Verfahren nach einem der vorhergehenden Ansprüche, wobei die zwei Sondengruppen von Nukleinsäuren derselben Art umfasst werden, zum Beispiel Oligonukleotid-Sonden.

11. Das Verfahren nach einem der vorhergehenden Ansprüche, wobei die zwei Sonden der zwei Gruppen einen 3'-radioaktiven Indikator oder einen 5'-radioaktiven Indikator umfassen, oder sie umfassen in ihrer Sequenz radioaktive Nukleotide, wobei die zwei die zwei Sonden der zwei Gruppen mehr bevorzugt einen 3'-radioaktiven Indikator umfassen.

12. Das Verfahren nach einem der vorhergehenden Ansprüche, wobei die erste und zweite radioaktive Markierung verschiedene Emissionsenergiespektra hat.

13. Das Verfahren nach Anspruch 12, wobei die erste Sondengruppe mit Tritium markiert und die zweite Sondengruppe mit einem radioaktiven Isotop markiert ist, ausgewählt aus ³⁵S, ³³P_{,} ³²P und ¹²⁵I.

14. Das Verfahren nach einem der vorhergehenden Ansprüche, wobei die zwei Sondengruppen gleichzeitig oder nacheinander mit der biologischen Probe in Kontakt gebracht werden und/oder die Zielnukleinsäuren durch gleichzeitiges Auswerten der *in situ* Hybridbildung zwischen den zwei Sondengruppen und der Probe nachgewiesen werden.

15. Das Verfahren nach einem der vorhergehenden Ansprüche, wobei im Wesentlichen dieselbe Menge der zwei Sondengruppen verwendet wird.

16. Das Verfahren nach einem der vorhergehenden Ansprüche, wobei die biologische Probe eine Säugetiergewebeprobe, vorzugsweise ein Gewebeschnitt ist.

17. Das Verfahren nach einem der vorhergehenden Ansprüche, wobei mehrere biologische Proben nebeneinander in Kontakt gebracht werden.

18. Das Verfahren nach einem der vorhergehenden Ansprüche, wobei die Proben auf eine oder mehrere Unterlage(n), vorzugsweise Glasunterlagen abgestellt werden.

19. Ein Verfahren für das gleichzeitige Nachweisen von Zielnukleinsäuren in mehreren biologischen Proben, umfassend:
a) das Bereitstellen biologischer Proben auf eine oder mehrere Unterlage(n), vorzugsweise Glasunterlagen,
b) das nebeneinander In-Kontakt-Bringen jeder der biologischen Proben auf der (den) Unterlage(n) mit mindestens zwei Gruppen radioaktiver Sonden, wobei die Sonden der ersten Gruppe spezifisch für eine erste Zielnukleinsäure sind und mit einem ersten radioaktiven Element markiert sind, und wobei die Sonden der zweiten Gruppe spezifisch für eine zweite Zielnukleinsäure sind und mit einem zweiten radioaktiven Element markiert sind, wobei die mindestens zwei Sondengruppen eine spezifische Zerfallsaktivität haben, die sich nicht mehr als ungefähr um das dreifache, vorzugsweise nicht mehr als ungefähr um das zweifache voneinander unterscheiden, und
c) das gleichzeitige Nachweisen der ersten und zweiten Zielnukleinsäuren in jeder der biologischen Proben durch *in situ* Auswerten der Hybridbildung zwischen den Sonden und den Proben.

20. Ein Verfahren für das gleichzeitige Nachweisen von Zielnukleinsäuren in einer biologischen Probe, wobei die biologische Probe nebeneinander mit den folgenden mindestens zwei Sondengruppen in Kontakt gebracht wird:
a) Sonden einer ersten Gruppe, die spezifisch für eine erste Zielnukleinsäure sind und mit einer ersten radioaktiven Markierung markiert sind, und Sonden einer zweiten Gruppe, die spezifisch für eine zweite Zielnukleinsäure sind und mit einer zweiten radioaktiven Markierung markiert sind,
b) Sonden der ersten Gruppe, die spezifisch für die erste Zielnukleinsäure sind und mit der zweiten radioaktiven Markierung markiert sind, und Sonden der zweiten Gruppe, die spezifisch für die zweite Zielnukleinsäure sind und mit der ersten radioaktiven Markierung markiert sind,
wobei die mindestens zwei Sondengruppen eine spezifische Zerfallsaktivität haben, die sich nicht mehr als ungefähr um das dreifache, vorzugsweise nicht mehr als ungefähr um das zweifache voneinander unterscheiden, und wobei das Verfahren ferner das gleichzeitige *in situ* Auswerten der Hybridbildung zwischen den Sonden und den Proben umfasst.

21. Ein Verfahren für das gleichzeitige Vergleichen der Zielgenexpression in mindestens zwei biologischen Proben, umfassend:
a) das nebeneinander In-Kontakt-Bringen biologischer Proben mit mindestens zwei Gruppen radioaktiver Sonden, wobei die Sonden der ersten Gruppe spezifisch für eine erste Zielnukleinsäure sind und mit einem ersten radioaktiven Element markiert sind, und wobei die Sonden der zweiten Gruppe spezifisch für eine zweite Zielnukleinsäure sind und mit einem zweiten radioaktiven Element markiert sind, wobei die mindestens zwei Sondengruppen eine spezifische Zerfallsaktivität haben, die sich nicht mehr als ungefähr um das dreifache, vorzugsweise nicht mehr als ungefähr um das zweifache voneinander unterscheiden,
b) das gleichzeitige *in situ* Auswerten der Hybridbildung zwischen den Sonden und den Proben, und
c) das quantitative Vergleichen der Zielgenexpression in den Proben durch gleichzeitiges Vergleichen der relativen Menge der Hybride, die sich unter den Proben gebildeten haben.

22. Das Verfahren nach einem der vorhergehenden Ansprüche, wobei eine der Sondengruppen spezifisch für eine Kontrollreferenznukleinsäure ist.

23. Das Verfahren nach einem der vorhergehenden Ansprüche, wobei das Auswerten der Hybridbildung Folgendes umfasst: (i) das Waschen der nicht-gebundenen Sonde und (ii) das Nachweisen von Radioaktivität auf der Probe.

24. Die Verwendung von zwei radioaktiven Sonden mit verschiedenen Nukleinsäuresequenzen und verschiedenen radioaktiven Markierungen, wobei die zwei Sonden eine spezifische Zerfallsaktivität haben, die sich nicht mehr als ungefähr um das dreifache, vorzugsweise nicht mehr als ungefähr um das zweifache voneinander unterscheiden, für die gleichzeitige *in vitro* oder *ex vivo* Genexpressionsanalyse auf einer gleichen biologischen Probe nach einem der Ansprüche 1 bis 26 der vorliegenden Anmeldung.

25. Ein Verfahren nach einem der Ansprüche 1 bis 23, ferner umfassend das In-Kontakt-Bringen der biologischen Probe(n) mit einer nicht-radioaktiven Sonde und/oder einem Affinitätsreagenz, um zusätzliche Zielnukleinsäure(n), Polypeptid(e) oder zelluläre Komponente(n) nachzuweisen.

26. Ein Verfahren für den gleichzeitigen Nachweis oder gleichzeitige Quantifizierung von mindestens zwei Zielpolypeptiden in einer Zelle oder einem Gewebe in mehreren biologischen Proben, umfassend:
a) das nebeneinander In-Kontakt-Bringen der biologischen Proben mit mindestens zwei Gruppen verschieden radioaktiv-markierter Antikörper, wobei die Antikörper der ersten Gruppe spezifisch für ein erstes Zielpolypeptid sind und mit einem ersten radioaktiven Element markiert sind, und wobei die Antikörper einer zweiten Gruppe spezifisch für ein zweites Zielpolypeptid sind und mit einem zweiten radioaktiven Element markiert sind, wobei die mindestens zwei Antikörpergruppen eine spezifische Zerfallsaktivität haben, die sich nicht mehr als ungefähr um das dreifache, vorzugsweise nicht mehr als ungefähr um das zweifache voneinander unterscheiden,
b) das *in situ* Auswerten der Hybridbildung zwischen den Antikörpern und den Proben, und gegebenenfalls
c) das quantitative Vergleichen der Zielpolypeptidexpression in den Proben durch Vergleichen der relativen Menge der Hybride, die sich unter den Proben gebildeten haben.

## Revendications

1. Méthode de détection simultanée d'acides nucléiques cibles dans un échantillon biologique, comprenant:
a) la mise en contact de l'échantillon biologique avec au moins deux séries de sondes radioactives, les sondes de la première série étant spécifiques d'un premier acide nucléique cible et marquée avec un premier radiomarqueur, et les sondes de la deuxième série étant spécifiques d'un deuxième acide nucléique cible et marquée avec un deuxième radiomarqueur, les au moins deux séries de sondes ayant une activité de désintégration spécifique ne differant pas par plus de trois fois l'une par rapport à l'autre, de préférence pas par plus de deux fois, et
b) la détection simultanée desdits premier et deuxième acides nucléiques cibles dans l'échantillon biologique en évaluant in situ la formation des hybrides entre la sonde et l'échantillon.

2. Méthode selon la revendication 1, **caractérisé en ce que** les deux séries de sondes ont une activité de désintégration spécifique comprise entre 5.10⁷ et 5.10¹⁰ cpm/µg.

3. Méthode selon la revendication 2, **caractérisé en ce que** les deux séries de sondes ont une activité de désintégration spécifique comprise entre 10⁸ et 10¹⁰ cpm/µg, de façon préférée entre 5.10⁸ et 5.10⁹ cpm/µg.

4. Méthode selon l'une quelconque des revendications précédentes, **caractérisé en ce que** les sondes sont des molécules d'ADN longues de 15 à 2000 paires de bases, de façon préférée longue de 15 à 500 paires de bases.

5. Méthode selon la revendication 4, **caractérisé en ce que** les sondes sont des oligonucléotides d'ADN simple-brins longs de 15 à 500 bases.

6. Méthode selon l'une quelconque des revendications 1 à 3, **caractérisé en ce que** les sondes sont des molécules d'ARN, longues de 15 à 3000 bases.

7. Méthode selon l'une quelconque des revendications précédentes, **caractérisé en ce que** les sondes sont marquées par un traceur radioactif en 3', de préférence une queue d'acide nucléique radiomarqué en 3', longue de 5-100.

8. Méthode selon l'une quelconque des revendications 1 à 7, **caractérisé en ce que** les sondes sont marquées par un traceur radioactif en 5'.

9. Méthode selon l'une quelconque des revendications 1 à 8, **caractérisé en ce que** les sondes comprennent, dans leur séquence, des nucléotides radioactifs.

10. Méthode selon l'une quelconque des revendications précédentes, **caractérisé en ce que** les deux séries de sondes sont composées d'acides nucléiques de même nature, par exemple des sondes oligonucléotidiques.

11. Méthode selon l'une quelconque des revendications précédentes, **caractérisé en ce que** les sondes des deux séries comprennent un traceur radioactif en 3' ou un traceur radioactif en 5' ou comprennent, dans leur séquence, des nucléotides radioactifs, les sondes des deux séries comprennent de façon préférée un traceur radioactif en 3'.

12. Méthode selon l'une quelconque des revendications précédentes, **caractérisé en ce que** le premier et le second radiomarqueurs ont un spectre d'énergie d'émission différent.

13. Méthode selon la revendication 12, **caractérisé en ce que** la première série de sondes est marquée avec le tritium et la deuxième série de sondes est marquée avec un radioisotope sélectionné parmi ³⁵S, ³³P, ³²P et ¹²⁵I.

14. Méthode selon l'une quelconque des revendications précédentes, **caractérisé en ce que** les deux séries de sondes sont mises en contact simultanément ou séquentiellement avec l'échantillon biologique et/ou les acides nucléiques cibles sont détectés par évaluation simultanée in situ de la formation des hybrides entre les deux séries de sondes et l'échantillon.

15. Méthode selon l'une quelconque des revendications précédentes, **caractérisé en ce qu'**essentiellement la même quantité des deux séries de sondes est utilisées.

16. Méthode selon l'une quelconque des revendications précédentes, **caractérisé en ce que** l'échantillon biologique est un échantillon de tissu de mammifère, de préférence une coupe de tissu.

17. Méthode selon l'une quelconque des revendications précédentes, **caractérisé en ce que** plusieurs échantillons biologiques sont mis en contact en parallèle.

18. Méthode selon l'une quelconque des revendications précédentes, **caractérisé en ce que** les échantillons sont déposés sur un ou plusieurs supports, de préférence un support en verre.

19. Méthode de détection simultanée d'acides nucléiques cibles dans plusieurs échantillons biologiques, comprenant:
a) la fourniture des échantillons biologiques sur un ou plusieurs supports, de préférence un support en verre,
b) la mise en contact, en parallèle, de chaque échantillon biologique sur le(s) support(s) avec au moins deux séries de sondes radioactives, les sondes de la première série étant spécifiques d'un premier acide nucléique cible et marquée avec un premier radioélément, et les sondes de la deuxième série étant spécifiques d'un deuxième acide nucléique cible et marquée avec un deuxième radioélément, les au moins deux séries de sondes ayant une activité de désintégration spécifique ne différant pas par plus de trois fois l'une par rapport à l'autre, de préférence pas par plus de deux fois, et
c) la détection simultanée desdits premier et deuxième acides nucléiques cibles dans chaque échantillon biologique en évaluant in situ la formation des hybrides entre les sondes et les échantillons.

20. Méthode de détection simultanée d'acides nucléiques cibles dans un échantillon biologique, **caractérisé en ce que** l'échantillon biologique est mis en contact, en parallèle avec au moins les deux séries de sondes suivantes:
a) des sondes d'une première série spécifiques d'un premier acide nucléique cible et marquées avec un premier radiomarqueur et des sondes d'une deuxième série spécifiques d'un deuxième acide nucléique cible et marquées avec un deuxième radiomarqueur,
b) des sondes de la première série spécifiques du premier acide nucléique cible et marquées avec le deuxième radiomarqueur et des sondes de la deuxième série spécifiques du deuxième acide nucléique cible et marquées avec le premier radiomarqueur,
**caractérisé en ce qu'**au moins deux séries de sondes ont une activité de désintégration spécifique ne différant pas par plus de trois fois l'une par rapport à l'autre, de préférence pas par plus de deux fois, et **en ce que** la méthode comprend en outre l'évaluation simultanée in situ de la formation d'hybrides entre les sondes et les échantillons.

21. Méthode pour comparer simultanément l'expression d'un gène cible dans au moins deux échantillons biologiques, comprenant:
a) la mise en contact, en parallèle, des échantillons biologiques avec au moins deux séries de sondes radioactives, les sondes de la première série étant spécifiques d'un premier acide nucléique cible et marquées avec un premier radioélément, et les sondes de la deuxième série étant spécifiques d'un deuxième acide nucléique cible et marquées avec un deuxième radioélément, les au moins deux séries de sondes ayant une activité de désintégration spécifique ne différant pas par plus de trois fois l'un par rapport à l'autre, de préférence pas par plus de deux fois,
b) l'évaluation simultanée in situ de la formation d'hybrides entre les sondes et les échantillons, et
c) la comparaison quantitative de l'expression du gène cible dans ledit échantillon en comparant simultanément la quantité relative d'hybrides formés entre les échantillons.

22. Méthode selon l'une quelconque des revendications précédentes, **caractérisé en ce qu'**une des séries de sondes est spécifique d'un acide nucléique contrôle de référence.

23. Méthode selon l'une des revendications précédentes, **caractérisé en ce que** l'évaluation de la formation d'hybride comprend (i) le lavage des sondes non liées et (ii) la détection de la radioactivité de l'échantillon.

24. Utilisation de deux sondes radioactives avec différentes séquences d'acide nucléique et différents marqueurs radioactifs, les deux sondes ayant des activités de désintégrations spécifiques ne différant pas par plus de trois fois l'un par rapport à l'autre, de préférence pas par plus de deux fois, pour une analyse simultanée de l'expression de gène in vitro ou ex vivo dans un même échantillon biologique selon l'une quelconque des revendications 1-26 de la demande sous-jacente.

25. Méthode selon l'une quelconque des revendications 1 à 23, comprenant en outre la mise en contact du(des) échantillon(s) biologique(s) avec une sonde non-radioactive et/ou un réactif d'affinité pour détecter un(des) acide(s) nucléiques(s) cible(s), polypeptide(s) ou composant(s) cellulaire(s).

26. Méthode pour la détection ou la quantification simultanée d'au moins deux polypeptides cibles d'une cellule ou d'un tissu, dans plusieurs échantillons biologiques, comprenant:
a) la mise en contact, en parallèle, des échantillons biologiques avec au moins deux séries d'anticorps différemment radiomarqués, les anticorps d'une première série étant spécifiques d'un premier polypeptide cible et marqués avec un premier radioélément, et les anticorps d'une deuxième série étant spécifiques d'un deuxième polypeptide cible et marqués avec un deuxième radioélément, les au moins deux séries d'anticorps ayant une activité de désintégration spécifique ne différant pas par plus de trois fois l'un par rapport à l'autre, de préférence pas par plus de deux fois,
b) l'évaluation in situ de la formation d'hybrides entre les anticorps et les échantillons, et facultativement
c) la comparaison quantitative de l'expression du polypeptide cible dans lesdits échantillons en comparant simultanément la quantité relative d'hybrides formés entre les échantillons.
